(19) Europäisches Patentamt
European Patent Office
Office européen des brevets

(11) Veröffentlichungsnummer: **0 335 831**
**A1**

(12) # EUROPÄISCHE PATENTANMELDUNG

(21) Anmeldenummer: **89810171.2**

(22) Anmeldetag: **07.03.89**

(51) Int. Cl.[4]: **C 07 D 277/56**
A 01 N 25/32

(30) Priorität: **15.03.88 CH 968/88 19.12.88 CH 4673/88**

(43) Veröffentlichungstag der Anmeldung:
**04.10.89 Patentblatt 89/40**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Nyffeler, Andreas, Dr.**
**Gründlerstrasse 4**
**CH-4312 Magden (CH)**

**Töpfl, Werner, Dr.**
**Dorneckstrasse 68**
**CH-4143 Dornach (CH)**

(54) **Thiazol-5-carbonsäureamide zum Schützen von Kulturpflanzen vor der phytotoxischen Wirkung von stark wirksamen Herbiziden.**

(57) Thiazol-5-carbonsäureamide der unten stehenden Formel I sind geeignet als Gegenmittel ("Antidot" oder "Safener") Kulturpflanzen, insbesondere Mais, Hirse, Reis, Getreide und Soja vor der phytotoxischen Wirkung von herbiziden Agrarchemikalien insbesondere Halogenacetaniliden zu schützen.

Die Thiazol-5-carbonsäureamide entsprechen der Formel I

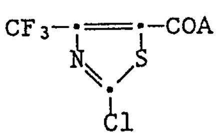

(I)

worin A einen Amidorest $NR_1R_2$ oder einen Hydrazidorest $-NR_3NR_4(CO)_mR_5$ bedeutet, worin m Null oder eins ist und $R_1$, $R_2$, $R_3$, $R_4$ und $R_5$, die in der Beschreibung gegebene Bedeutung haben.

EP 0 335 831 A1

## Beschreibung

### Thiazol-5-carbonsäureamide zum Schützen von Kulturpflanzen vor der phytotoxischen Wirkung von stark wirksamen Herbiziden

Die vorliegende Erfindung betrifft ein Mittel zum Schutz von Kulturpflanzen vor der phytotoxischen Wirkung von stark wirksamen Agrarchemikalien, insbesondere von Herbiziden, welches als Herbizid-antagonisierenden Wirkstoff ein Thiazol-5-carbonsäureamid enthält, ferner Mittel welche neben diesem antagonisierenden Wirkstoff bereits das Herbizid enthalten und ein Verfahren zur selektiven Unkrautbekämpfung mittels dieser Herbizide und diesem Gegenmittel.

Es ist bekannt, dass Herbizide aus den verschiedensten Stoffklassen, wie Triazine, Harnstoffderivate, Carbamate, Thiolcarbamate, Halogenacetanilide, Halogenphenoxyessigsäuren usw. bei der Anwendung in wirksamer Dosis gelegentlich neben den zu bekämpfenden Unkräutern auch die Kulturpflanzen in gewissem Masse schädigen. Es können klimatische Verhältnisse oder eine Bodenbeschaffenheit vorliegen, so dass die für normale Bedingungen empfohlene Herbizidmenge als Ueberdosis wirkt. Die Qualität des Saatgutes kann bei der Herbizidverträglichkeit auch eine Rolle spielen. Um diesem Problem zu begegnen, sind schon verschiedene Stoffe vorgeschlagen worden, welche befähigt sind, die schädigende Wirkung des Herbizides auf die Kulturpflanze spezifisch zu antagonisieren, d.h. die Kulturpflanze zu schützen, ohne dabei die Herbizidwirkung auf die zu bekämpfenden Unkräuter merklich zu beeinflussen. Dabei hat es sich gezeigt, dass die vorgeschlagenen Gegenmittel sowohl bezüglich der Kulturpflanzen als auch bezüglich des Herbizides und gegebenenfalls auch in Abhängigkeit von der Applikationsart oft sehr artspezifisch wirken, d.h. ein bestimmtes Gegenmittel eignet sich oft nur für eine bestimmte Kulturpflanze und einige wenige herbizide Stoffklassen.

Vom wirtschaftlichen Gesichtspunkt müssen bei der Verwendung von chemischen Schutzmitteln (Safener) gegen Herbizide mehrere Faktoren in Betracht gezogen werden. Das chemische Behandlungsmittel muss ein Stoff sein, der lange genug in enger Berührung mit dem Nutzpflanzengut bleibt, um die auflaufenden Nutzpflanzen zu schützen, bis keine phytotoxisch gefährdende Konzentration an dem Herbizid mehr vorhanden ist. Andere Verbindungen neigen dazu, phytotoxische Wirkungen auf die Nutzpflanzen selbst auszuüben. Dies kann auf dreierlei Weise erfolgen, nämlich durch Verhinderung des Auflaufens der Nutzpflanzen, so dass eine schlechte Keimung stattfindet, durch Erzeugung von Missbildungen beim Wachstum der auflaufenden Nutzpflanzen und durch Verkümmerung oder Verzögerung des Wachstums der Nutzpflanzen. In einigen Fällen lassen sich diese Wirkungen durch einfaches Herabsetzen der Anwendungskonzentration der Mittel beseitigen. In anderen Fällen scheint es aber nicht möglich zu sein, die Verbindungen in einer genügend niedrigen Konzentration anzuwenden, dass die Schädigung auf ein wirtschaftlich tragbares Ausmass herabgesetzt wird. In einigen Fällen schützen die chemischen Behandlungsmittel die Nutzpflanzen zwar gegen die herkömmlichen Mengen an Herbiziden; wenn aber gelegentlich Ueberdosierungen vorkommen, wie z.B. beim Ueberlappen von Spritzflächen, bieten die Verbindungen keinen Schutz mehr. Für die technische Anwendung ist es daher in Anbetracht der Möglichkeit solcher gelegentlicher und unbeabsichtiger Ueberdosierungen z.B. in sich überlappender Behandlungsflächen wünschenswert, das Saatgut gegen etwa das Doppelte der beabsichtigten Anwendungskonzentration der Unkrautbekämpfung zu schützen.

Thiazol-5-carbonsäurederivate sind aus der Literatur bekannt. Im US Patent 3 725 427 werden 2,4-dimethylthiazol-5-carboxamide als Fungizide beschrieben; in den US Patenten 4 199 506, 4 251 261 sowie in den Europäischen Offenlegungsschriften EP-A 27 018, 44 201 und 64 353 sind 2-Chlor-4-trifluormethyl-thiazol-5-carbonsäurederivate als Gegenmittel (Antidots) zur Verminderung der phytotoxischen Wirkung von starken Herbiziden an Kulturpflanzen beschrieben worden.

Es wurde nun gefunden, dass sich überraschenderweise die Thiazol-5-carbonsäureamide der Formel I hervorragend dazu eignen, Kulturpflanzen gegen die schädigende Wirkung von Agrarchemikalien, wie z.B. Halogenacyl-Herbi ziden zu schützen. Die Thiazol-5-carbonsäureamide werden daher im folgenden Text auch mit "Gegenmittel", "Antidot" oder "Safener" bezeichnet.

Die Thiazol-5-carbonsäureamide entsprechen der Formel I

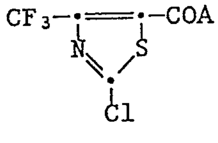

(I)

worin A einen Rest $-NR_1R_2$ oder $-NR_3-NR_4(CO)_mR_5$, $R_1$, $R_2$, $R_3$, $R_4$ und $R_5$ unabhängig voneinander je Wasserstoff, $C_1$-$C_8$Alkyl oder $C_3$-$C_8$Cycloalkyl, welches unsubstituiert oder durch $C_1$-$C_8$Alkoxy, $C_2$-$C_8$Alkoxyalkoxy, $C_1$-$C_8$Alkylthio, Cyano, einen Rest $-COOR_6$, $C_1$-$C_4$Alkylcarbamoyl, Di-$C_1$-$C_4$alkylcarbamoyl, $C_1$-$C_4$Alkylamino, Di-$C_1$-$C_4$Alkylamino, Piperidinocarbonyl, Pyrrolidinocarbonyl, Piperidino oder Pyrrolidino substituiert ist; ferner $C_3$-$C_8$Alkenyl oder $C_3$-$C_8$Cycloalkenyl, welches unsubstituiert oder substituiert ist durch Halogen, $C_1$-$C_8$ Alkoxy, $C_3$-$C_8$Cycloalkenyl, oder einen Rest Cyan, $-COOR_6$, $C_1$-$C_4$Alkylcarbamoyl, Di-$C_1$-$C_4$alkylcarbamoyl, U, Pyrrolidino-carbamoyl oder Piperidinocarbamoyl substituiert ist, ferner $C_3$-$C_8$Alkinyl, welches unsubstituiert oder durch U substituiert ist, ferner die Reste $-(E)_m-U$ oder $-(E)_m-Q$; $R_1$ und $R_2$ resp. $R_4$ und $R_5$ zusammen mit dem Stickstoffatom, an das sie gebunden sind, bilden auch einen gesättigten

oder ungesättigten 5 - 9 gliedrigen Heterocyclus, der ein-oder mehrfach durch Sauerstoff, Schwefel, Stickstoff, -NH-, -N$C_1$-$C_4$Alkyl, -CO- oder -C(0$R_7$)0$R_8$ und durch Halogen, Cyan, $C_1$-$C_8$Alkoxy, Amino, $C_1$-$C_4$Alkylamino, Di-$C_1$-$C_4$-Alkylamino, oder einen Rest -COO$R_6$ substituiert sein kann; $R_6$ ist Wasserstoff, $C_1$-$C_8$Alkyl, $C_3$-$C_8$Cycloalkyl, $C_3$-$C_8$Alkenyl, $C_3$-$C_8$Cycloalkenyl, $C_3$-$C_8$Alkinyl, $C_2$-$C_8$Alkoxyalkyl, $C_4$-$C_8$Alkoxy-alkoxyalkyl, $C_1$-$C_4$Halogenalkyl, -($C_1$-$C_3$Alkyl)$_m$U, -($C_1$-$C_3$Alkyl)$_m$Q, $C_1$-$C_4$Halogenalkoxy, $C_1$-$C_4$Halogenalkoxy $C_1$-$C_4$alkyl;
$R_7$ und $R_8$ sind unabhängig voneinander je $C_1$-$C_4$Alkyl oder
$R_7$ und $R_8$ bilden zusammen eine 2 - 4 gliedrige Alkylenkette, U ist ein Phenyl- oder Naphthylrest, der unsubstituiert oder ein- oder mehrfach durch Halogen, $C_1$-$C_4$Alkyl, -Y-$C_1$-$C_4$Alkyl, $C_1$-$C_4$Halogenalkyl, $C_1$-$C_4$Halogenalkoxy, Cyano, Nitro, -COOH, -COO$R_7$, -CON$H_2$, -CONH$R_7$, -CON$(R_7)_2$, $SO_2$NH$R_7$, $SO_2N(R_7)_2$, $SO_2NH_2$, Pyrrolidino, Piperidino, Pyrrolidinocarbonyl oder Piperidinocarbonyl substituiert ist.

E ist eine $C_1$-$C_8$Alkylen, $C_2$-$C_8$Alkenylenkette, die unsubstituiert oder durch Halogen, $C_1$-$C_4$Alkoxy, $C_1$-$C_4$Alkylthio, $C_1$-$C_4$ Halogenalkoxy oder einen Rest -CO(O)$_m$$R_6$, -(CO)$_m$N$H_2$, -(CO)$_m$NH$R_7$, -(CO)$_m$N$R_7$$R_8$, -(CO)$_m$Q substituiert und/oder durch ein Glied -CO- oder -C(O$R_7$)O$R_8$- unterbrochen ist,
Q ist ein gesättigter oder ungesättigter 5 - 12 gliedriger Heterocyclus, der 1 - 4 Heteroatome oder eine Sulfinyl- oder Sulfonylgruppe auch in Kombination mit 1 - 2 Heteroatomen enthalten kann, der durch eine oder zwei Carbonylgruppen unterbrochen und benzanneliert sein kann und m ist Null oder 1.

Wegen ihrer herbizid-antagonistischen Wirkung sind diejenigen Thiazol-5-carbonsäureamide hervorzuheben, welche der Formel Ia entsprechen

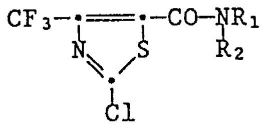
(Ia)

worin die Reste $R_1$ und $R_2$ unabhängig voneinander je Wasserstoff oder $C_1$-$C_8$-Alkyl oder $C_3$-$C_8$Cycloalkylrest, welcher unsubstituiert oder durch Halogen, Cyano, $C_1$-$C_4$Alkoxy oder -COO$R_6$ substituiert ist, einen $C_2$-$C_8$Alkenylrest, der unsubstituiert oder durch Halogen, Cyano, $C_1$-$C_4$Alkoxy oder -COO$R_6$ substituiert ist oder einen $C_3$-$C_8$Alkinylrest,
$R_2$ ferner einen Rest -(E)$_m$U oder -(E)$_m$Q,
$R_1$ und $R_2$ zusammen eine 4 - 5 gliedrige Alkylenkette, die durch Sauerstoff, Schwefel, -NH- oder -N$R_7$ unterbrochen und/oder durch $C_1$-$C_4$Alkyl substituiert sein kann,
E $C_1$-$C_4$Alkylen, $C_3$-$C_4$-Cycloalkylen, $C_2$-$C_4$Alkenylen oder $C_2$-$C_4$Alkinylen, U Phenyl unsubstituiert oder durch Halogen, Cyano, $C_1$-$C_4$Alkyl, $C_1$-$C_4$Alkoxy, $C_1$-$C_4$Haloalkyl, $C_1$-$C_4$Haloalkoxy, COO$R_6$, -$SO_2NH_2$, -$SO_2$NH$R_7$, -$SO_2N(R_7)_2$, -CON$H_2$, -CONH$R_7$, -CON$(R_7)_2$ substituiert,
Q einen 5 - 6 gliedrigen Heterocyclus der unsubstituiert oder durch Halogen, $C_1$-$C_4$Alkyl, $C_1$-$C_4$Alkoxy, $C_1$-$C_4$Haloalkyl oder $C_1$-$C_4$Haloalkoxy substituiert ist und $R_6$ und $R_7$ die unter der Formel I gegebene Bedeutung haben.

Dabei sind diejenigen Amide besonders aktiv, in denen $R_1$ Wasserstoff, $C_1$-$C_8$Alkyl oder $C_3$-$C_8$Alkinyl und $R_2$ $C_1$-$C_8$Cyanoalkyl oder $C_3$-$C_8$Cyanocycloalkyl, $C_3$-$C_8$Alkinyl, Phenyl, Halophenyl, $C_1$-$C_4$Alkylphenyl, $C_1$-$C_4$Alkylhalophenyl, Phenylamino, Halophenylamino oder Furyl bedeuten, insbesondere diejenigen, in denen der Rest -N$R_1$$R_2$, -N-Methyl-N-1 cyanopentylamin oder -N-Methyl-N-(2,6-dichlorbenzyl)amin und $R_7$ bedeuten.

Ebenfalls als sehr aktive Herbizid-Antagonisten sind die Thiazol-5-carbonsäure-hydrazide der Formel Ib

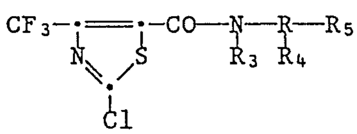
(Ib)

worin $R_3$, $R_4$ und $R_5$ unabhängig voneinander je Wasserstoff, $C_1$-$C_8$Alkyl, $C_3$-$C_8$Cycloalkyl, Phenyl oder Benzyl bedeuten, wobei der Phenylring unsubstituiert oder durch Halogen, $C_1$-$C_4$Alkyl, $C_1$-$C_4$ Haloalkyl, $C_1$-$C_4$Alkoxy, $C_1$-$C_4$Haloalkoxy, -COO$R_6$, -CON$H_2$, -CONH$R_7$, -CON$(R_7)_2$, -SON$H_2$, SONH$R_7$, -$SO_2N(R_7)_2$, substituiert ist und $R_4$ und $R_5$ bilden zusammen auch eine 4 - 5 gliedrige Alkylenkette, die durch Sauerstoff, Schwefel, -NH- oder -N$R_7$ unterbrochen und/oder durch $C_1$-$C_4$ Alkyl substituiert sein kann.

Soweit optisch isomere Verbindungen der Formel I existieren, sind im Rahmen der vorliegenden Erfindung sowohl die optisch reinen Isomere wie auch die Isomerengemische zu verstehen.

Unter Halogen selbst in den Definitionen sowie Halogen als Teil in Halogenalkoxy, Halogenalkyl oder Halogenalkenyl sind Fluor, Chlor, Brom und Jod, vorzugsweise jedoch Fluor, Chlor und Brom, insbesondere aber Chlor zu verstehen.

Unter Alkoxy ist zu verstehen: Methoxy, Aethoxy, n-Propyloxy, i-Propyloxy oder die isomeren Butyloxy-, Pentyloxy-, Hexyloxy-, Heptyloxy- oder Octyloxyreste, insbesondere aber Methoxy, Aethoxy oder i-Propyloxy.

Beispiele für ungesättigte Substituenten oder Substituententeile sind Allyl, Allyloxy, Propargyl, Propargyloxy, Methallyl, Methallyloxy, Butenyl, Butenyloxy, Butinyl, Butinyloxy, 3,3,3-Trifluor-1-propenyl, 3,3,3-Trichlor-1-propinyl oder 2,3-Dichlorpropen-1-yl.

Alkoxyalkylreste werden repräsentiert durch Methoxymethyl, Aethoxymethyl, Methoxyäthyl und Aethoxyäthyl, Propoxyäthyl, Isopropoxyäthyl, Butoxyäthyl, Allyloxyäthyl, insbesondere aber Methoxyäthyl. Halogenalkyl als Substituenten wie Halogenalkoxy oder Halogenalkylthio steht in der Regel für Chlormethyl, Fluormethyl, Difluormethyl, Trifluormethyl, Dichlormethyl, Trichlormethyl, 2-Chloräthyl, 2,2,2-Trifluoräthyl, 1,1,2,2-Tetrafluoräthyl, Pentafluoräthyl, 1,1,2-Trifluor-2-chloräthyl, 2,2,2,-Trifluor-1,1-dichloräthyl, Pentachloräthyl, 3,3,3-Trifluorpropyl, 2,3-Dichlorpropyl, 2-Chlorpropyl, 1,1,2,3,3,3-Hexafluorpropyl, 3,3,3-Trichlorpropyl, 2,2,2-Trichloräthyl, 1-Chloräthyl, insbesondere aber Chlormethyl, Dichlormethyl, Trichlormethyl und 1-Chloräthyl.

In diesen Definitionen werden unter Alkylresten Reste mit 1 bis 18 Kohlenstoffatomen verstanden. Diese Reste können geradkettig oder verzweigt sein. Die gebräuchlichsten Reste sind beispielsweise Methyl, Aethyl, n-Propyl, Isopropyl, n-Butyl, Isobutyl, sek.Butyl, tert.Butyl, n-Pentyl, Isopentyl, n-Hexyl und n-Octyl. Die Alkenyl- und Alkinylreste können ebenfalls geradkettig oder verzweigt sein und umfassen 3 bis 18 Kohlenstoffatome. Die am verbreitetsten Reste sind beispielsweise Allyl, Methallyl, Buten, Butadien, Propinyl, Methylpropinyl, 1-Butinyl, 2-Butinyl. Cycloalkyl- oder Cycloalkenylreste haben vorzugsweise 3 - 12 Kohlenstoffatome, sie können auch benzanneliert sein. Typische Vertreter sind beispielsweise Cyclopropyl, Cyclopentyl, Cyclohexyl, Cyclohexenyl, Indan, Tetrahydronaphthalin, Decalin. Unter Halogen wird Fluor, Chlor, Brom, Jod, insbesondere Fluor und Chlor verstanden. Halogenalkyl- und Halogenalkenyl-Reste können ein- oder mehrfach mit Halogen substituiert sein.

Die obgenannten Reste können unsubstituiert oder substituiert sein. Typische Substituenten dieser Reste sind beispielsweise die Halogenatome, über Sauerstoff, Schwefel oder eine Iminogruppe gebundene Alkyl-, Alkenyl-, Alkinyl-, oder Cycloalkyl-, Aryl- oder Aralkylreste, die ihrerseits wieder substituiert sein können. Diese Substituenten können aber auch über eine Sulfinyl-, Sulfonyl-, Carbonyl-, Carbonyloxy, Carbonylthio, Carbamoyl-, Sulfamoyl- oder eine Amino-oxybrücke an die alicyclischen Kohlenwasserstoffreste gebunden sein.

Der Substituent Q aber auch die Reste $R_3$ und $R_4$ können zusammen mit dem Stickstoffatom, an das sie gebunden sind, auch einen gesättigten oder ungesättigten 5 bis 12-gliedrigen Heterocyclus bilden, der noch ein, zwei oder drei weitere Heteroatome oder eine Sulfinyl- resp. Sulfonylgruppe enthält, durch ein oder zwei Carbonylgruppen unterbrochen sein kann, und welcher benzannelliert, unsubstituiert oder substituiert sein kann.

Als Heteroatome kommen dabei ein, zwei oder drei weitere Stickstoffatome, bis zwei Schwefel- oder Sauerstoffatome in Frage, wobei 2 Sauerstoffatome nicht direkt benachbart sein können

Beispiele für solche Heterocyclen sind untenstehend aufgeführt: Pyrrolin, Pyrrolidin, Imidazolin, Imidazolidin, Pyrazolin, Pyrazolidin, Isoxazolin, Isoxazolidin, Oxazolin, Oxazolidin, Isothiazolidin, Thiazoline, Thiazolidine, Dithiazolidine, Oxadiazolidine, Piperidin, Piperazin, Tetrahydropyrimidin und -pyrazin, Morpholin, Thiomorpholin, Thiazine, Hexahydrotriazine, Tetrahydrotriazine, Oxadiazine, Oxatriazine, Hexahydroazepin, Hexahydrodiazepine, Diazepine, Hexahydrooxazepine, Azacyclooctan, Indolin, Isoindolin, Benzimidazolin, Benzindazolin, Benzoxazolin, Benzthiazoline, Benzisoxazolin, Benztriazol, Tetrahydrochinolin, Tetrahydroisochinolin, Tetrahydrochinazolin, Tetrahydrochinoxalin, Tetrahydrophtalazin, Benzmorpholin, Benzothiomorpholin, Tetrahydrobenzazepine und Tetrahydrodiazepine, Tetrahydrobenzoxazepine, 1,5-diazabicyclo[4,3,0]-nonane, Dihydrobenzoxazine, 1,6-diazabicyclo[5,3,0]decane, 1,4-diazabicyclo[3,3,0]octane, 1,5-diazabicyclo[4,4,0]decane.

Oben erwähnte Heterocyclen können auch die Bedeutung von Substituenten haben. Weitere Beispiele von heterocyclischen Systemen mit Substituentenfunktion sind beispielsweise Pyrrol, Imidazol, Pyrazol, Isoxazol, Oxazol, Isothiazol, Thiazol, Triazole, Oxadiazole, Thiadiazole, Tetrazole, Oxatriazole, Thiatriazole, Furan, Tetrahydrofuran, Dioxole, Dioxolane, Oxathiole, Oxathiolane, Thiophen, Tetrahydrothiophen, Dithiolane, Dithiazole, Pyridin, Pyrane, Thiopyrane, Pyridazin, Pyrimidin, Pyrazin, Tetrahydropyran, Tetrahydrothiopyran, Dioxine, Dioxane, Dithiine, Dithiane, Oxazine, Thiazine, Oxathiine, Oxathiane, Triazine, Oxadiazine, Thiadiazine, Oxathiazine, Dioxazine, Azepine, Oxepine, Thiepine, Diazepine, Oxazepine, Indole, Benzofurane, Benzothiophene, Indazole, Benzimidazole, Benzdioxole, Benzdithiole, Benzisoxazole, Benzthiazole, Benzoxazole, Benzoxathiole, Benztriazole, Benzoxadiazole, Benzofurazan, Benzothiadiazole, Chinolin, Isochinolin, Chromene, Chroman, Isochromen, Isochroman, Thiochromene, Isothiochromene, Thiochroman, Isothiochroman, Cinnolin, Chinazolin, Chinoxalin, Phtalazin, Benzdioxine, Benzdithiine, Benzoxazine, Benzdioxane, Benzoxathiane, Benzotriazine, Benzazepine, Benzdilazepine, Benzoxazepine, Purine, Pteridine, Phenoxazine, Phenothiazine.

Die heterocyclischen Reste können wie oben erwähnt substituiert sein.

Einige Verbindungen der Formel I sind in der Europäischen Patentanmeldung EP-A 279 239 beschrieben. Sie können nach bekannten Methoden hergestellt werden.

Die Thiazol-5-carbonsäureamide der Formel I werden z.B. gemäss USP 4,199,506 hergestellt, indem man einen Acrylsäureester der Formel II mit Chlorcarbonyl-sulfenylchlorid der Formel III umsetzt, entsprechend der Gleichung

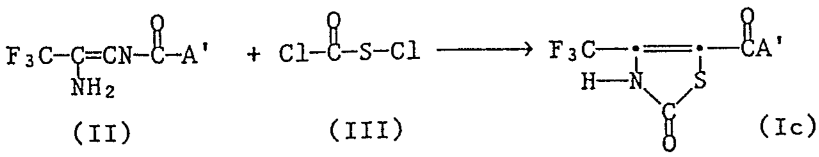

(II) (III) (Ic)

Das entstandene 2-Oxo-4-trifluormethylthiazol-5-carbonsäurederivat wird mit Phosphoroxychlorid behandelt, wobei ein 2-Chlor-4-trifluormethylthiazol-5-carbonsäurederivat der Formel I entsteht:

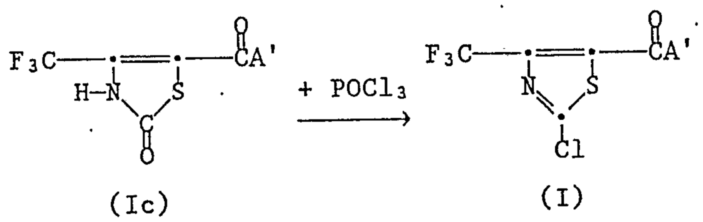


(Ic) (I)

Die Acrylsäurederivate der Formel III können gemäss J. Het. Chem. 9 (1972) 513 hergestellt werden, oder indem man einen Acetoessigessigester mit Trifluormethylnitril umsetzt in Gegenwart von Natriumacetat in siedendem Lösungsmittel

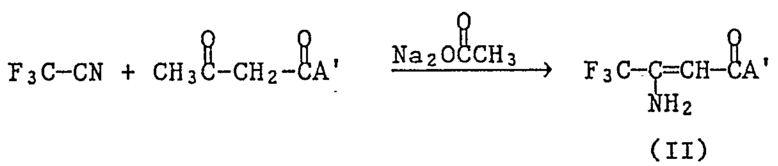


(II)

Ausgehend vom 2-Chlor-4-trifluormethylthiazol-5-carbonsäurechlorid können nach bekannten Methoden und entsprechend dem folgenden Reaktionsschema weitere Wirkstoffe der Formel I hergestellt werden:

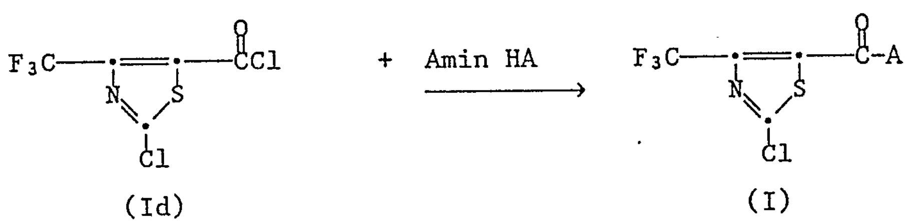


(Id) (I)

In diesen Formeln haben A und $R_1$ die oben gegebene Bedeutung.

In diesen Umsetzungen werden in Anspassung an die jeweiligen Reaktionsbedingungen inerte Lösungs- und Verdünnungsmittel verwendet. Als Beispiele sind zu nennen:

Halogenkohlenwasserstoffe, insbesondere Chlorkohlenwasserstoffe, wie Tetrachlorethylen, Tetrachlorethan, Dichlorpropan, Methylenchlorid, Dichlorbutan, Chloroform, Chlornaphthalin, Dichlornaphthalin, Tetrachlorkohlenstoff, Trichlorethan, Trichlorethylen, Pentachlorethan, Difluorbenzol, 1,2-Dichlorethan, 1,1-Dichlorethan, 1,2-cis-Dichlorethylen, Chlorbenzol, Fluorbenzol, Brombenzol, Jodbenzol, Dichlorbenzol, Dibrombenzol, Chlortoluol, Trichlorbenzol; Ether, wie Ethylpropylether, Methyl-tert.-butylether, n-Butylethylether, Di-n-butylether, Di-isobutylether, Diisoamylether, Diisopropylether, Anisol, Phenetol, Cyclohexylmethylether, Diethylether, Ethylenglykoldimethylether, Tetrahydrofuran, Dioxan, Thioanisol, Dichlordiethylether; Nitrokohlenwasserstoffe wie Nitromethan, Nitroethan, Nitrobenzol, Chlornitrobenzol, o-Nitrotoluol; Nitrile wie Acetonitril, Butyronitril, Isobutyronitril, Benzonitril, m-Chlorbenzonitril; aliphatische oder cycloaliphatische Kohlenwasserstoffe, wie Heptan, Pinan, Nonan, Cymol, Benzinfraktionen innerhalb eines Siedepunktintervalles von 70° bis 190° C, Cyclohexan, Methylcyclohexan, Dekalin, Petrolether, Hexan, Ligroin, Trimethylpentan, Trimethylpentan, 2,3,3-Trimethylpentan, Octan; Ester wie Ethylacetat, Acetessigester, Isobutylacetat; Amide, z.B. Formamid, Methylformamid, Dimethylformamid.

Die erfindungsgemässen Thiazol-carbonsäureamide der Formel 1 zeigen ausgezeichnete Safenerwirkung und sind in ihrer Wirkung bekannten Verbindungen überlegen.

Ein Gegenmittel oder Antidot der Formel I kann je nach Anwendungszweck zur Vorbehandlung des Saatgutes der Kulturpflanze (Beizung des Samens oder der Stecklinge) eingesetzt oder vor oder nach der Saat in den Boden gegeben werden. Es kann aber auch für sich allein oder zusammen mit dem Herbizid vor oder nach dem Auflaufen der Pflanzen appliziert werden. Die Behandlung der Pflanze oder des Saatgutes mit

dem Antidot kann daher grundsätzlich unabhängig vom Zeitpunkt der Applikation der phytotoxischen Chemikalie erfolgen. Die Behandlung der Pflanze kann jedoch auch durch gleichzeitige Applikation von phytotoxischer Chemikalie und Gegenmittel (Tankmischung) erfolgen. Die preemergente Behandlung schliesst sowohl die Behandlung der Anbaufläche vor der Aussaat (ppi = pre plant incorporation) als auch die Behandlung der angesäten, aber noch nicht bewachsenen Anbauflächen ein.

Die Aufwandmengen des Gegenmittels im Verhältnis zum Herbizid richten sich weitgehend nach der Anwendungsart. Bei einer Feldbehandlung, bei der Herbizid und Gegenmittel entweder gleichzeitig (Tankmischung) oder separat appliziert werden, liegt das Verhältnis der Mengen von Gegenmittel zu Herbizid im Bereich von 1:100 bis 5:1. In der Regel wird bei einem Mengenverhältnis von Gegenmittel zu Herbzid von 1:1 bis 1:50 die volle Schutzwirkung erreicht. Bei der Samenbeizung und ähnlichen gezielten Schutzmassnahmen werden jedoch weit geringere Mengen Gegenmittel im Vergleich mit den später pro Hektar Anbaufläche verwendeten Mengen an Herbizid benötigt. Im allgemeinen werden bei der Samenbeizung pro kg Samen 0,1-10 g Gegenmittel benötigt. In der Regel wird mit 0,1-5 g Gegenmittel pro kg Samen bereits die volle Schutzwirkung erreicht. Falls das Gegenmittel kurz vor der Aussaat durch Samenquellung appliziert werden soll, so werden zweckmässig Lösungen des Gegenmittels verwendet, welche den Wirkstoff in einer Konzentration von 1-10'000 ppm enthalten. In der Regel wird mit Konzentrationen des Gegenmittels von 100-1'000 ppm die volle Schutzwirkung erreicht.

Die zur Erzielung der gewünschten Safening Effekts notwendige Menge des Antidots der Formel I kann je nach Anwendungsart und Zeitpunkt sehr unterschiedlich sein und ist durch einfache Versuche nicht zu ermitteln.

In der Regel liegt zwischen protektiven Massnahmen, wie Samenbeizung und Behandlung von Stecklingen mit einem Gegenmittel der Formel I und der möglichen späteren Feldbehandlung mit Agrarchemikalien ein längerer Zeitraum. Vorbehandeltes Saat- und Pflanzengut kann später in der Landwirtschaft, im Gartenbau und in der Forstwirtschaft mit unterschiedlichen Chemikalien in Berührung kommen. Die Erfindung bezieht sich daher auf protektive Mittel für Kulturpflanzen, die als Wirkstoff ein Gegenmittel der Formel I zusammen mit üblichen Trägerstoffen enthalten. Solche Mittel können gegebenenfalls zusätzlich jene Agrarchemikalien enthalten, vor deren Einfluss die Kulturpflanze geschützt werden soll.

Als Kulturpflanzen gelten im Rahmen vorliegender Erfindung alle Pflanzen, die in irgendeiner Form Ertragsstoffe, wie Samen, Wurzeln, Stengel, Knollen, Blätter, Blüten, ferner Inhaltsstoffe, wie Oele, Zucker, Stärke, Eiweiss usw., produzieren und zu diesem Zweck angebaut werden. Zu diesen Pflanzen gehören beispielsweise sämtliche Getreidearten, wie Weizen, Roggen, Gerste und Hafer, daneben vor allem Kulturhirse, Mais, Baumwolle, Zuckerrüben, Zuckerrohr, Soja, Bohnen und Erbsen und insbesondere Reis.

Das Gegenmittel kann überall dort eingesetzt werden, wo eine Kulturpflanze der vorgenannten Art vor der schädlichen Wirkung einer Agrarchemikalie geschützt werden soll. Dabei kommen als Agrarchemikalien in erster Linie Herbizide der verschiedensten Stoffklassen in Betracht.

Hervorragende Schutzwirkung gegen Sulfonylharnstoffherbizide, gegen Aryloxyphenoxypropionsäureherbizide und gegen Chloracetanilidherbizide werden bei Anwendung der Antidotes der Formel I besonders in Mais, Sorghum und Reis beobachtet.

Sulfonylharnstoffherbizide, deren schädigende Wirkung gegenüber Kulturpflanzen mit Hilfe der Thiazol-5-carbonsäureamide der Formel I vermindert oder aufgehoben werden kann, sind in letzter Zeit in grosser Zahl bekannt geworden. Aus der Vielzahl der Publikationen, welche sich der Offenbarung von herbizid wirksamen Sulfonylharnstoffderivaten widmen, sollen beispielhaft das US-Patent 4 127 405 sowie die publizierten Europäischen Patentanmeldungen EP-A-7687, EP-A-30142, EP-A-44807, EP-A-44808, EP-A-51466, EP-A-70802, EP-A-84020, EP-A-87780, EP-A-102925, EP-A-108708, EP-A-120814, EP-A-136061, EP-A-184385, EP-A-206995 und EP-A-237292 genannt sein.

Typische Vertreter von herbiziden Sulfonylharnstoffderivaten sind unter der Formel IV

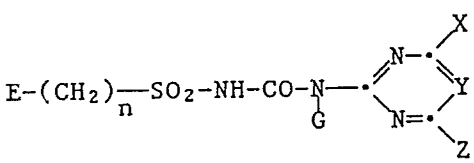

(IV)

zusammengefasst, worin

E eine Gruppe

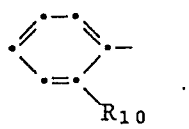 , 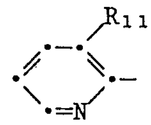 , 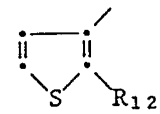 ,

oder 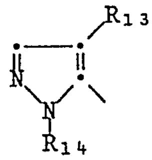 ,

n die Zahl null oder eins,
G Wasserstoff oder Methyl,
X Methoxy, Aethoxy, Difluormethoxy, Methyl oder Chlor
Y CH oder H,
Z Methoxy, Methyl, Difluormethoxy, Cyclopropyl oder Methylamino,
$R_{10}$ $C_2$-$C_5$-Alkoxyalkoxy, $C_1$-$C_4$-Halogenalkoxy, $C_1$-$C_4$-Halogenalkylthio, $C_2$-$C_4$-Halogenalkenyl, Chlor oder $C_1$-$C_4$-Alkoxycarbonyl,
$R_{11}$ Trifluormethyl oder Di($C_1$-$C_4$-alkyl)carbamoyl,
$R_{12}$ $C_1$-$C_4$-Alkoxycarbonyl,
$R_{13}$ $C_1$-$C_4$-Alkoxycarbonyl, und
$R_{14}$ $C_1$-$C_4$-Alkyl bedeuten.

Unter die Formel IV fallen folgende herbizide Einzelwirkstoffe:
N-(3-Trifluormethylpyridin-2-ylsulfonyl)-N′-(4,6-dimethoxypyrimidin-2-yl)-harnstoff,
N-(3-Dimethylcarbamoylpyridin-2-ylsulfonyl)-N′-(4,6-dimethoxypyrimidin-2-yl)-harnstoff,
N-(1-Methyl-4-äthoxycarbonylpyrazol-2-ylsulfonyl) -N′-(4,6-dimethoxypyrimidin-2-yl)-harnstoff,
N-(2-Methoxycarbonylthien-3-ylsulfonyl)-N′-(4-methoxy-6-methyl-1,3,5-triazin-2-yl)-harnstoff,
N-(2-Methoxycarbonylbenzylsulfonyl)-N′-(4,6-dimethoxypyrimidin-2-yl)-harnstoff,
N-(2-Methoxycarbonylphenylsulfonyl)-N′-(4,6-bis-difluormethoxypyrimidin-2-yl)-harnstoff,
N-(2-Methoxycarbonylphenylsulfonyl)-N′-(4-äthoxy-6-methylamino-1,3,5-triazin-2-yl)-harnstoff,
N-(2-Methoxycarbonylphenylsulfonyl)-N′-(4-methoxy-6-methyl-1,3,5-triazin-2-yl)-harnstoff,
N-(2-Aethoxycarbonylphenylsulfonyl)-N′-(4-chlor-6-methoxypyrimidin-2-yl)-harnstoff,
N-(2-Methoxycarbonylphenylsulfonyl)-N′-(4-methoxy-6-methyl-1,3,5-triazin-2-yl)-N′-methylharnstoff,
N-(2-Methoxycarbonylphenylsulfonyl) -N′-(4,6-dimethoxypyrimidin-2-yl)-harnstoff,
N-(2-Chlorphenylsulfonyl)-N′-(4-methoxy-6-methyl-1,3,5-triazin-2-yl)-harnstoff,
N-[2-(2-Chloräthoxy)-phenylsulfonyl]-N′-(4-methoxy-6-methyl-1,3,5-triazin-2-yl)-harnstoff, und
N-[ 2-(2-Methoxyäthoxy)-phenylsulfonyl]-N′-(4,6-dimethoxy-1,3,5-triazin-2-yl)-harnstoff.

Halogenacetanilide, deren schädigende Wirkung gegenüber Kulturpflanzen mit Hilfe der Thiazol-5-carbonsäureamide der Formel I vermindert oder aufgehoben werden kann, sind ebenfalls bereits in grosser Zahl bekannt geworden. Solche Halogenacetanilide können durch die folgende allgemeine Formel V beschrieben werden:

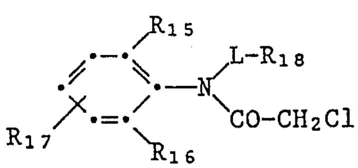 (V)

worin
L eine $C_1$-$C_4$-Alkylenbrücke,
$R_{15}$ $R_{16}$ und $R_{17}$ unabhängig voneinander Wasserstoff, Halogen, $C_1$-$C_4$-Alkyl, $C_1$-$C_4$-Alkoxy, $C_1$-$C_4$-Halogenalkyl, $C_2$-$C_5$-Alkoxyalkyl oder $C_2$-$C_5$-Alkylthioalkyl, und
$R_{18}$ $C_1$-$C_4$-Alkoxy, -COOH, $C_1$-$C_4$-Alkoxycarbonyl, -$CONH_2$, $C_1$-$C_4$-Alkylcarbamoyl, Di-$C_1$-$C_4$-alkylcarbamoyl, Cyan, $C_1$-$C_4$-Alkylcarbonyl,
gegebenenfalls substituiertes Benzoyl,
gegebenenfalls substituiertes Furyl,
gegebenenfalls substituiertes Thienyl,
gegebenenfalls substituiertes Pyrrolyl,
gegebenenfalls substituiertes Pyrazolyl,
gegebenenfalls substituiertes 1,3,4-Oxadiazol-2-yl,
gegebenenfalls substituiertes 1,3,4-Thiadiazol-2-yl,
gegebenenfalls substituiertes 1,2,4-Triazol-3-yl,
gegebenenfalls substituiertes Dioxolanyl,

gegebenenfalls substituiertes Dioxanyl,
gegebenenfalls substituiertes 1,3,4-Triazol-2-yl oder
gegebenenfalls substituiertes Tetrahydrofuryl, bedeutet.

Unter die Formel V fallen insbesondere die folgenden herbiziden Chloracetanilidderivate:
N-Aethoxymethyl-N-chloracetyl-2-äthyl-6-methylanilin,
N-Chloracetyl-N-methoxymethyl-2,6-diäthylanilin,
N-Chloracetyl-N-(2-methoxyäthyl)-2,6-dimethylanilin,
N-Chloracetyl-N-(2-n-propoxyäthyl)-2,6-dimethylanilin,
N-Chloracetyl-N-(2-isopropoxyäthyl)-2,6-dimethylanilin,
N-Chloracetyl-N-(2-methoxyäthyl)-2-äthyl-6-methylanilin,
N-Chloracetyl-N-(methoxyäthyl)-2,6-diäthylanilin,
N-(2-Aethoxyäthyl)-N-chloracetyl-2-äthyl-6-methylanilin,
N-Chloracetyl-N-(2-methoxy-1-methyläthyl)-2-methylanilin,
N-Chloracetyl-N-(2-methoxy-1-methyläthyl)-2,6-dimethylanilin,
N-Chloracetyl-N-(2-methoxy-1-methyläthyl)-2,6-diäthylanilin,
N-Chloracetyl-N-(2-methoxy-1-methyläthyl)-2-äthyl-6-methylanilin,
N-(2-Aethoxyäthyl)-N-chloracetyl-2,6-diäthylanilin,
N-Chloracetyl-N-(2-n-propoxyäthyl)-2-äthyl-6-methylanilin,
N-Chloracetyl-N-(2-n-propoxyäthyl)-2,6-diäthylanilin,
N-Chloracetyl-N-(2-isopropoxyäthyl)-2-äthyl-6-methylanilin,
N-Aethoxycarbonylmethyl-N-chloracetyl-2,6-dimethylanilin
N-Aethoxycarbonylmethyl-N-chloracetyl-2,6-diäthylanilin
N-Chloracetyl-N-methoxycarbonylmethyl-2,6-dimethylanilin,
N-Chloracetyl-N-(2,2-diäthoxyäthyl)-2,6-dimethylanilin,
N-Chloracetyl-N-(2-methoxy-1-methyläthyl)-2,3-dimethylanilin,
N-(2-Aethoxyäthyl)-N-chloracetyl-2-methylanilin,
N-Chloracetyl-N-(2-methoxyäthyl)-2-methylanilin,
N-Chloracetyl-N-(2-methoxy-2-methyläthyl)-2,6-dimethylanilin,
N-(2-Aethoxy-2-methyläthyl)-N-chloracetyl-2-äthyl-6-methylanilin,
N-Chloracetyl-N-(1-äthyl-1-methoxyäthyl)-2,6-dimethylanilin,
N-Chloracetyl-N-(2-methoxyäthyl)-2-methoxy-6-methylanilin,
N-n-Butoxymethyl-N-chloracetyl-2-tert.-butylanilin,
N-(2-Aethoxyäthyl-2-methyläthyl)-N-chloracetyl-2,6-dimethylanilin,
N-Chloracetyl-N-(2-methoxyäthyl)-2-chlor-6-methylanilin,
N-(2-Aethoxyäthyl)-N-chloracetyl-2-chlor-6-methylanilin,
N-(2-Aethoxyäthyl)-N-chloracetyl-2,3,6-trimethylanilin,
N-Chloracetyl-1-(2-methoxyäthyl)-2,3,6-trimethylanilin,
N-Chloracetyl-N-cyanomethyl-2,6-dimethylanilin,
N-Chloracetyl-N-(1,3-dioxolan-2-ylmethyl)-2 6-dimethylanilin,
N-Chloracetyl-N-(1,3-dioxolan-2-ylmethyl)-2-äthyl-6-methylanilin,
N-Chloracetyl-N-(1,3-dioxan-2-ylmethyl)-2-äthyl-6-methylanilin,
N-Chloracetyl-N-(2-furylmethyl)-2,6-dimethylanilin,
N-Chloracetyl-N-(2-furylmethyl)-2-äthyl-6-methylanilin,
N-Chloracetyl-N-(2-tetrahydrofurylmethyl)-2,6-dimethylanilin,
N-Chloracetyl-N-(N,N-dimethylcarbamoylmethyl)-2,6-dimethylanilin,
N-(n-Butoxymethyl)-N-chloracetyl-2,6-diäthylanilin,
N-( 2-n-Butoxyäthyl)-H-chloracetyl-2,6-diäthylanilin,
N-Chloracetyl-N-( 2-methoxy-1,2-dimethyläthyl)-2,6-dimethylanilin,
N-Chloracetyl-N-isopropyl-2,3-dimethylanilin,
N-Chloracetyl-N-isopropyl-2-chloranilin,
N-Chloracetyl-N-(1H-pyrazol-1-ylmethyl)-2,6-dimethylanilin,
N-Chloracetyl-N-(1H-pyrazol-1-ylmethyl)-2-äthyl-6-methylanilin,
N-Chloracetyl-N-(1H-1,2,4-triazol-1-ylmethyl)-2,6-dimethylanilin,
N-Chloracetyl-N-(1H-1,2,4-triazol-1-ylmethyl)-2,6-diäthylanilin,
N-Benzoylmethyl-N-chloracetyl-2,6-diäthylanilin,
N-Benzoylmethyl-N-chloracetyl-2-äthyl-6-methylanilin,
N-Chloracetyl-N-(5-methyl-1,3,4-oxadiazol-2-yl) -2,6-diäthylanilin,
N-Chloracetyl-N-(5-methyl-1,3,4-oxadiazol-2-yl)-2-äthyl-6-methylanilin,
N-Chloracetyl-N-(5-methyl-1,3,4-oxadiazol-2-yl)-2-tert.butylanilin,
N-Chloracetyl-N-(4-chlorbenzoylmethyl)-2,6-dimethylanilin und
N-Chloracetyl-N-(1-methyl-5-methylthio-1,3,4-triazol-2-ylmethyl)-2,6-diäthylanilin.

Weitere Halogenacetanilide, deren schädigende Wirkung auf Kulturpflanzen durch die Thiazol-5-carbonsäureamide der Formel I vermindert oder aufgehoben werden kann, sind in R. Wegler, Chemie der Pflanzenschutz- und Schädlingsbekämpfungsmittel, Bd. 8, Seiten 90-93 und Seiten 322-327 aufgeführt.

Aryloxyphenoxypropionsäureherbizide, deren schädigende Wirkung gegenüber Kulturpflanzen mit Hilfe der

Thiazol-5-carbonsäureamide der Formel I vermindert oder aufgehoben werden kann, sind in grosser Zahl bekannt. Solche Aryloxyphenoxypropionsäurederivate können durch die folgende allgemeine Formel VI beschrieben werden:

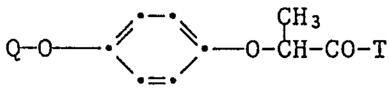 (VI)

worin

Q für den Rest

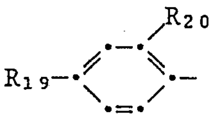 , 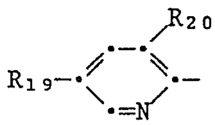 , 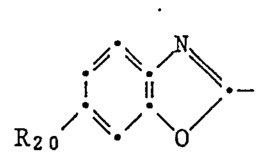 ,

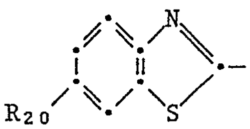 oder 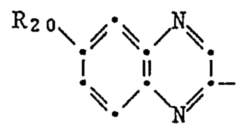

und T für

$-NR_{21}R_{22}$, $-N(CN)R_{21}$, $-OR_{22}$, $SR_{22}$ oder $-O-N{=}CR_{23}R_{22}$ stehen, wobei

$R_{19}$ Halogen oder Trifluormethyl,

$R_{20}$ Wasserstoff oder Halogen,

$R_{21}$ und $R_{22}$ unabhängig voneinander Wasserstoff, $C_1$-$C_8$-Alkoxy, $C_1$-$C_8$Alkyl, Phenyl oder Benzyl, $R_{21}$ und $R_{22}$ zusammen mit dem sie tragenden Stickstoffatom einen 5- bis 6-gliedrigen gesättigten Stickstoffheterocyclus, der ein Sauerstoff- oder Schwefelatom enthalten kann,

$R_{23}$ $C_1$-$C_4$-Alkyl, $C_3$-$C_4$-Alkenyl, $C_3$-$C_4$-Alkinyl oder $C_2$-$C_4$-Alkoxyalkyl

$R_{24}$ Wasserstoff oder das Kation-Aequivalent eines Alkalimetalls, Erdalkalimetalles von Kupfer oder Eisen; einem quaternären $C_1$-$C_4$-Alkylammonium- oder $C_1$-$C_4$-Hydroxyalkylammonium-Rest; einen gegebenenfalls ein- oder mehrfach durch Amino, Halogen, Hydroxyl, Cyan, Nitro, Phenyl, $C_1$-$C_4$-Alkoxy, Polyäthoxy mit 2 bis 6 Aethylenoxideinheiten, $-COOR_{27}$, $-COSR_{27}$, $-CONH_2$, $-CON(C_1$-$C_4$-alkoxy)-$C_1$-$C_4$-alkyl, -CO-N-di-$C_1$-$C_4$-alkyl, CONH-$C_1$-$C_4$-alkyl, $-N(C_1$-$C_4$-alkoxy)-$C_1$-$C_4$-alkyl oder Di-$C_1$-$C_4$-alkylamino substituierten $C_1$-$C_9$-Alkylrest; einen gegebenenfalls durch Halogen oder $C_1$-$C_4$-Alkoxy substituierten $C_3$-$C_9$-Alkenylrest; einen gegebenenfalls durch Halogen oder $C_1$-$C_4$-Alkoxy substituierten $C_3$-$C_9$-Alkinylrest; $C_3$-$C_9$-Cycloalkyl; oder gegebenenfalls durch Cyan, $C_1$-$C_4$-Alkyl, $C_1$-$C_4$-Alkoxy, Acetyl, $-COOR_{27}$, $COSR_{27}$, $-CONH_2$, $-CON(C_1$-$C_4$-alkoxy)-$C_1$-$C_4$-alkyl, -CO-N-di-$C_1$-$C_4$-alkyl oder -CONH-$C_1$-$C_4$-alkyl substituiertes Phenyl,

$R_{25}$ und $R_{26}$ unabhängig voneinander $C_1$-$C_4$-Alkyl oder zusammen eine 3- bis 6-gliedrige Alkylenkette und $R_{27}$ Wasserstoff, $C_1$-$C_6$-Alkyl, $C_1$-$C_6$-Halogenalkyl, $C_2$-$C_6$-Alkoxyalkyl, $C_3$-$C_6$-Alkenyl, $C_3$-$C_6$-Halogenalkyl, $C_3$-$C_6$-Alkinyl oder $C_3$-$C_6$-Halogenalkinyl bedeuten.

Unter die Formel VI fallen insbesondere die folgenden herbiziden Aryloxyphenoxypropionsäurederivate:
2-[4-(3,5-Dichlorpyridin-2-yloxy)-phenoxy]-propionsäure-propargylester,
2-[4-(3,5-Dichlorpyridin-2-yloxy)-phenoxy]-thiopropionsäure-propargylester,
2-[4-(5-Chlor-3-fluorpyridin-2-yloxy)-phenoxy]-propionsäure-methylester,
2-[4-(5-Chlor-fluorpyridin-2-yloxy)-phenoxy]-propionsäure-butylester,
2-[4-(5-Trifluormethylpyridin-2-yloxy)-phenoxy]-propionsäure-butylester,
2-[4-(5-Trifluormethylpyridin-2-yloxy)-phenoxy]-propionsäure-methylester,
2-[4-(6-Chlorchinoxalin-2-yloxy)-phenoxy]-propionsäure-äthylester und
2-[4-(6-Chlorbenzoxazolin-2-yloxy)-phenoxy]-propionsäure-äthylester.

Die Antidotes der Formel I eignen sich ganz besonders, um Kulturpflanzen vor den herbiziden Wirkungen der Herbizide der Formeln IV, V, VI zu schützen.

Agrochemische Mittel, welche in einer gemeinsamen Formulierung neben dem Antidote der Formel I ein Sulfonylharnstoffherbizid, ein Chloracetanilidherbizid oder ein Aryloxyphenoxypropionsäureherbizid enthalten sind zum Einsatz als selektive Herbizide in Nutzpflanzenkulturen geeignet. Vorzugsweise enthalten die erfindungsgemässen herbiziden Mittel neben einem Antidote der Formel I einen Sulfonylharnstoff der Formel IV, Chloracetanilid der Formel V oder ein Aryloxyphenoxypropionsäurederivat der Formel VI.

Die angewendete Menge an Gegenmittel, sofern sie nicht auf die Samen gebeizt wird, schwankt zwischen etwa 0,01 und etwa 5 Gewichtsteilen pro Gewichtsteil Herbizid. In der Praxis ermittelt man jeweils, von Fall zu Fall, das heisst je nach verwendetem Herbizid-Typ, welches Verhältnis in Bezug auf optimale Wirkung bei der speziellen Kulturpflanze das geeignetste ist.

Die Erfindung betrifft auch ein Verfahren zur selektiven Bekämpfung von Unkräutern in Kulturpflanzenbeständen, wobei die Kulturpflanzenbestände, Teile der Kulturpflanzen oder Anbauflächen für Kulturpflanzen mit einem Herbizid und einer Verbindung der Formel I oder einem Mittel, welches diese Kombination enthält, behandelt werden. Die die Herbizid/Antidot-Kombination enthaltenden Mittel bilden ebenfalls einen Bestandteil der vorliegenden Erfindung.

Bei den zu bekämpfenden Unkräutern kann es sich sowohl um monokotyle wie um dikotyle Unkräuter handeln.

Für die Verwendung von Verbindungen der Formel I oder sie enthaltender Mittel zum Schützen von Kulturpflanzen gegen schädigende Wirkungen von Agrarchemikalien kommen verschiedene Methoden und Techniken in Betracht, wie beispielsweise die folgenden:

i) Samenbeizung

a) Beizung der Samen mit einem als Spritzpulver formulierten Wirkstoff durch Schütteln in einem Gefäss bis zur gleichmässigen Verteilung auf der Samenoberfläche (Trockenbeizung). Man verwendet dabei etwa 10 bis 500 g Wirkstoff der Formel I (bei einer 25 %igen Formulierung: 40 g bis 2 kg Spritzpulver) pro 100 kg Saatgut.

b) Beizung der Samen mit einem Emulsionskonzentrat des Wirkstoffs oder mit einer wässrigen Lösung des als Spritzpulver formulierten Wirkstoffs der Formel I nach Methode a) (Nassbeizung).

c) Beizung durch Tauchen des Saatguts in einer Brühe mit 50-3200 ppm Wirkstoff der Formel I während 1 bis 72 Stunden und gegebenenfalls nachfolgendes Trocknen der Samen (Tauchbeizung, Seed soaking).

Die Beizung des Saatguts oder die Behandlung des angekeimten Sämlings sind naturgemäss die bevorzugten Methoden der Applikation, weil die Wirkstoffbehandlung vollständig auf die Zielkultur gerichtet ist. Man verwendet in der Regel 10 g bis 500 g, vorzugsweise 50 bis 400 g Aktivsubstanz pro 100 kg Saatgut, wobei man je nach Methodik, die auch den Zusatz anderer Wirkstoffe oder Mikronährstoffe ermöglicht, von den angegebenen Grenzkonzentrationen nach oben oder unten abweichen kann (Wiederholungsbeize).

ii) Applikation aus Tankmischung

Eine flüssige Aufarbeitung eines Gemisches von Gegenmittel und Herbizid (gegenseitiges Mengenverhältnis zwischen 10:1 und 1:30) wird verwendet, wobei die Aufwandmenge an Herbizid 0,001 bis 10 kg pro Hektar beträgt. Solche Tankmischung wird vorzugsweise vor oder nach der Aussaat appliziert oder 5 bis 10 cm tief in den noch nicht gesäten Boden eingearbeitet.

iii) Applikation in die Saatfurche

Das Gegenmittel wird als Emulsionskonzentrat, Spritzpulver oder als Granulat in die offene besäte Saatfurche eingebracht und hierauf wird nach dem Decken der Saatfurche in normaler Weise das Herbizid im Vorauflaufverfahren appliziert.

iv) Kontrollierte Wirkstoffabgabe

Der Wirkstoff wird in Lösung auf mineralische Granulatträger oder polymerisierte Granulate (Harnstoff/Formaldehyd) aufgezogen und trocknen gelassen. Gegebenenfalls kann ein Ueberzug aufgebracht werden (Umhüllungsgranulate), der es erlaubt, den Wirkstoff über einen bestimmten Zeitraum dosiert abzugeben.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Di-oktylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer

Natur wie insbesondere Dolomit oder zerkleinerte Pflanzenrückstände verwendet werden.

Als oberflächenaktive Verbindungen kommen je nach Art des zu formulierenden Wirkstoffes der Formel I nichtionogene, kation-und/oder anionaktive Tenside in guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie zum Beispiel die Na-oder K-Salze der Oel- oder Stearinsäure, oder von natürlichen Fettsäuregemischen, die beispielsweise aus Kokosnuss- oder Talgöl gewonnen werden können, genannt. Ferner sind auch die Fettsäuremethyltaurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, zum Beispiel das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8-22 Kohlenstoffatomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenolpolyäthoxyäthanol, Polyäthylenglykol und Oktylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 Kohlenstoffatomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, beispielsweise das Stearyltrimethylammoniumchlorid oder das Benzyldi(2-chloräthyl)äthylammoniumbromid.

Die in der Formulierungstechnik gebräuchlichen Tenside sind u.a. in folgenden Publikationen beschrieben:
"1985 International Mc Cutcheon's Emulsifiers & Detergents", Glen Rock NJ USA, 1985",
"H. Stache, "Tensid-Taschenbuch", 2. Aufl., C. Hanser Verlag München, Wien 1981,
M. and J. Ash. "Encyclopedia of Surfactants", Vol. I-III, Chemical Publishing Co., New York, 1980-1981.

Die agrochemischen Zubereitungen enthalten in der Regel 0,1 bis 95 %, insbesondere 0,1 bis 80 % Wirkstoff der Formel I, 1 bis 99,9 % eines festen oder flüssigen Zusatzstoffes und 0 bis 25 % insbesondere 0,1 bis 25 % eines Tensides.

Insbesondere setzen sich bevorzugte Formulierungen folgendermassen zusammen: (% = Gewichtsprozent)

Emulgierbare Konzentrate

| | | | |
|---|---|---|---|
| Aktives Wirkstoffgemisch | 1 bis 20 %, | bevorzugt | 5 bis 10 % |
| oberflächenaktives Mittel: | 5 bis 30 %, | vorzugsweise | 10 bis 20 % |
| flüssiges Trägermittel: | 50 bis 94 %, | vorzugsweise | 70 bis 85 % |

Stäube:

| | | | |
|---|---|---|---|
| Aktives Wirkstoffgemisch: | 0,1 bis 10 %, | vorzugsweise | 0,1 bis 1 % |
| festes Trägermittel: | 99,9 bis 90 %, | vorzugsweise | 99,9 bis 99 %. |

Suspension-Konzentrate

| | | | |
|---|---|---|---|
| Aktives Wirkstoffgemisch: | 5 bis 75 %, | vorzugsweise | 10 bis 50 % |
| Wasser: | 94 bis 25 %, | vorzugsweise | 88 bis 30 % |
| oberflächenaktives Wasser: | 1 bis 40 %, | vorzugsweise | 2 bis 30 % |

Benetzbare Pulver

| | | | |
|---|---|---|---|
| Aktives Wirkstoffgemisch: | 0,5 bis 90 %, | vorzugsweise | 1 bis 80 % |
| oberflächenaktives Mittel: | 0,5 bis 20 %, | vorzugsweise | 1 bis 15 % |
| festes Trägermaterial: | 5 bis 95 %, | vorzugsweise | 15 bis 90 % |

Granulate

| | | | |
|---|---|---|---|
| aktives Wirkstoffgemisch: | 0,5 bis 30 %, | vorzugsweise | 3 bis 15 % |
| festes Trägermittel: | 99,5 bis 70 %, | vorzugsweise | 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,01 bis 10 kg AS/ha, vorzugsweise 0,25 bis 5 kg AS/ha.

Die Mittel können auch weitere Zusätze wie Stabilisatoren, Entschäumer, Konservierungmittel, Viskositätsregulatoren, Bindemittel, Haftmittel sowie Dünger oder andere Wirkstoffe zur Erzielung spezieller Effekte enthalten.

Die folgenden Beispiele sollen zur Erläuterung der Erfindung dienen, ohne diese einzuschränken. Temperaturen sind in den Beispielen und den nachfolgenden Tabellen in Centigraden angegeben, die Druckangaben sind in Millibar (mbar) angegeben, Prozent- und Teilangaben beziehen sich auf das Gewicht.

Beispiel 1: Herstellung von 2-Chlor-5-[N-(1-cyano-cyclopentyl)-N-methyl-carbamoyl]-3-trifluormethyl-thiazol

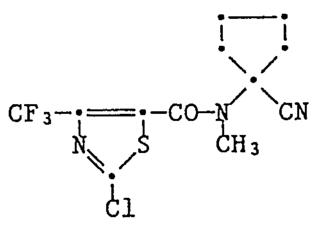

Eine Lösung von 112.5 g 2-Chlor-4-trifluormethyl-thiazol-5-carbonsäure-chlorid (0.45 Mole) werden in 200 ml Acetonitril vorgelegt und eine Mischung von 55.9 g 1-Cyano-1-methylamino-cyclopentan (0.45 Mole) und 46.0 g Triäthylamin (0.45 Mole) bei 0° zugetropft. Dabei fällt Aminhydrochlorid aus. Die Suspension wird über Nacht bei Raumtemperatur gerührt und dann in 2 l Wasser eingerührt. Das abgeschiedene Oel wird in Essigester aufgenommen, über Natriumsulfat getrocknet und das Lösungsmittel unter vermindertem Druck abdestilliert. Der Rückstand kristallisiert beim Verrühren mit Aether. Man erhält farblose Kristalle. Schmelzpunkt 134 - 136°: Ausbeute 125 g (83 % d. Th.).

In analoger Weise zu diesem Beispiel werden die in der Tabelle 1 aufgeführten Thiazol-5-carbonsäureamide hergestellt.

Tabelle 1

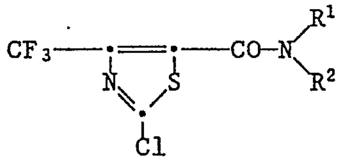

(Id)

| Nr. | $R^1$ | $R^2$ | Physikal.Daten |
|---|---|---|---|
| 1.001 | Allyl | Allyl | Smp. 38 - 40° |
| 1.002 | Allyl | 2-Methoxy-äthyl | |
| 1.003 | Allyl | Isopropyl | |
| 1.004 | 2-Methyl-2-propen-1-yl | Isopropyl | |
| 1.005 | 2-Methyl-2-propen-1-yl | Cyclohexyl | |
| 1.006 | 2-Chlor-2-propen-1-yl | 2-Methoxy-äthyl | |
| 1.007 | 2-Chlor-2-propen-1-yl | Isopropyl | |
| 1.008 | 2-Chlor-2-propen-1-yl | Cyclohexyl | Smp. 70 - 72° |
| 1.009 | 2-Chlor-2-propen-1-yl | 2-Chlor-2-propen-1-yl | Smp. 38 - 40° |
| 1.010 | 3-Chlor-2-propen-1-yl | Propyl | |
| 1.011 | Propyl | 2-Methoxy-äthyl | |
| 1.012 | Methyl | Cyclohexyl | |
| 1.013 | Methyl | Benzyl | $n_D^{20}$ 1.5408 |
| 1.014 | Methyl | 2,6-Dichlor-benzyl | Smp. 118 - 120° |
| 1.015 | Isopropyl | Benzyl | $n_D^{20}$ 1.5333 |
| 1.016 | Isopropyl | 4-Chlorbenzyl | |
| 1.017 | H | Benzyl | Smp. 102 - 105° |
| 1.018 | H | Phenyl | Smp. 137 - 138 |
| 1.019 | Methyl | Phenyl | Smp. 57 - 59° |
| 1.020 | Methyl | 4-Chlorphenyl | |
| 1.021 | Ethyl | 2-Chlor-4-brom-phenyl | |
| 1.022 | Ethyl | 3-Trifluormethyl-phenyl | |
| 1.023 | H | $-CH_2-COOC_2H_5$ | |
| 1.024 | Methyl | $-CH_2-COOCH_3$ | |
| 1.025 | Isopropyl | $-CH_2-CO-NH-C_3H_7(i)$ | |
| 1.026 | Cyclopropyl | $-CH_2-CO-N(C_2H_5)_2$ | |
| 1.027 | Ethyl | $-CH_2-CH_2-CN$ | |
| 1.028 | Allyl | $-CH_2-CH_2-CN$ | |
| 1.029 | Cyclohexyl | $-CH_2-CH_2-CN$ | |
| 1.030 | Phenyl | $-CH_2-CH_2-CN$ | |
| 1.031 | Phenyl | $-CH_2-CH_2-COOH$ | |
| 1.032 | Cyclohexyl | $-CH_2-CH_2-COOCH_3$ | Smp. 116 - 120° |
| 1.033 | 2,6-Dimethyl-phenyl | $-CH(CH_3)COOCH_3$ | Smp. 120 - 123° |
| 1.034 | Phenyl | $-CH_2-COOH$ | |
| 1.035 | Phenyl | $-CH_2-CN$ | |
| 1.036 | 4-Chlor-phenyl | $-CH_2-CN$ | |
| 1.037 | 2,4-Dichlorphenyl | $-CH_2-CN$ | |
| 1.038 | 3,4-Dichlorphenyl | $-CH_2-CN$ | Smp. 128 - 131° |
| 1.039 | 3-Trifluormethyl-phenyl | $-CH_2-CN$ | |
| 1.040 | Methyl | 1-Cyanocyclopent-1-yl | Smp. 130 - 133° |
| 1.041 | H | 3-Trifluoromethyl-cyclohexyl | |
| 1.042 | Pyrrolidino | | |
| 1.043 | Piperidino | | |
| 1.044 | 2-Methylpiperidino | | |

| Nr. | $R^1$ | $R^2$ | Physikal.Daten |
|---|---|---|---|
| 1.045 | 2-Ethylpiperidino | | |
| 1.046 | Hexahydroazepino | | |
| 1.047 | Morpholino | | |
| 1.048 | 2,2,5,5-Tetramethyl 1,3-oxazolidin-3-yl | | |
| 1.049 | 5,5-Dimethyl-2,2-tetra-methylen-1,3-oxazolidin-3-yl | | |
| 1.050 | 5,5-Dimethyl-2,2-pentamethylen-1,3-oxazolidin-3-yl | | |
| 1.051 | 2-Phenyl-1,3-oxazolidin-3-yl | | |
| 1.052 | 2,2-Tetramethylen-benzthiazol-3-yl | | |
| 1.053 | 2-Oxo-pyrrolidino | | |
| 1.054 | Hexahydro-2-oxo-azepino | | |
| 1.055 | 3-Oxo-thiomorpholino | | |
| 1.056 | 2-Oxo-1,3-oxazolin-3-yl | | |
| 1.057 | 2-Trichloromethyl-1,3-oxazolidin-3-yl | | |
| 1.058 | H | 2-Chlorbenzyl | Smp. 115 -116° |
| 1.059 | H | 2-Hexylbenzyl | |
| 1.060 | $C_2H_5$ | $C_2H_5$ | Smp. 40 - 41° |
| 1.061 | Allyl | H | Smp. 56 - 58° |
| 1.062 | Phenylethyl | H | Smp. 88 - 89° |
| 1.063 | $C_2H_5$ | 2,6-Dichlorbenzyl | Smp. 89 - 91° |
| 1.064 | 2-Chlorbenzyl | H | Smp. 115 - 116° |
| 1.065 | Propargyl | Propargyl | Smp. 100 - 101° |
| 1.066 | $C_4H_9$-n | 2,6-Dichlorbenzyl | $n_D^{20}$ 1.5491 |
| 1.067 | Allyl | Ethoxycroton-2-yl | Smp. 72 - 74° |
| 1.068 | $-CH_2-CH(CH_3)_2$ | 2-Chlorallyl | $n_D^{20}$ 1.5027 |
| 1.069 | 4-Chlor-2-fluor-6-iso-propoxyphenyl | H | Smp. 123 - 125° |
| 1.070 | Cyano-dimethylmethyl | Methoxyethyl | Smp. 130 - 132° |
| 1.071 | Chlorphenyl | H | Smp. 125 - 126° |
| 1.072 | Cyano-dimethyl-methyl | H | |
| 1.073 | 2,2-Dimethylindanyl | H | Smp. 174 - 175° |
| 1.074 | 3,5-Bis-trifluormethyl-phenyl | H | Smp. 125 - 127° |
| 1.075 | Diphenylmethyl | H | Smp. 177 - 179° |
| 1.076 | 2,6-Difluorphenyl | H | Smp. 160 - 161° |
| 1.077 | 5-Trifluormethyl-thiazol-2-yl | H Triethylammonium-Salz | Smp. 136 - 138° |
| 1.078 | 2-Carboxyl-4-chlorphenyl | H | Smp. 132 - 134° |
| 1.079 | 3-Trifluormethylcyclo-hexyl | H | Smp. 106 - 109° |
| 1.080 | 2,4,6-Trichloranilino | H | Smp. 184 - 186° |
| 1.081 | Furfuryl | H | Smp. 100 - 102° |
| 1.082 | 3,4-Methylendioxybenzyl | H | Smp. 129 - 131° |
| 1.083 | 4-Amidosulfonylphenyl | H | Smp. 186 - 189° |
| 1.084 | 1,2-Diphenyleth-1-yl | H | Smp. 146 - 148° |
| 1.085 | α-Methylbenzyl | H | Smp. 131 - 133° |
| 1.086 | 4-Fluorbenzyl | H | Smp. 128 - 130° |
| 1.087 | 2,2-Diphenyleth-1-yl | H | Smp. 127 - 129° |
| 1.088 | 1-Cyano-cyclopent-1-yl | Methoxycarbonylmethyl | Smp. 145 - 147° |
| 1.089 | 1-Cyano-cyclohex-1-yl | Methyl | Smp. 145 - 147° |

| Nr. | $R^1$ | $R^2$ | Physikal.Daten |
|---|---|---|---|
| 1.090 | 2-Methoxycarbonyl-4-chlorphenyl | H | |
| 1.091 | 2-Methylaminocarbonyl-4-chlorphenyl | H | |
| 1.092 | 2-Dimethylaminoethyl | H | |
| 1.193 | 1-Cyanocyclopent-1-yl | $CH_3$ | |
| 1.194 | Cyano-dimethyl-methyl | $CH_3$ | Smp. 78 - 80 |

Beispiel 2: Herstellung von 2-Chlor-4-trifluormethyl-thiazol-5-carbonsäure-N'-(2.4.6-trichlor-phenyl)-hydrazid

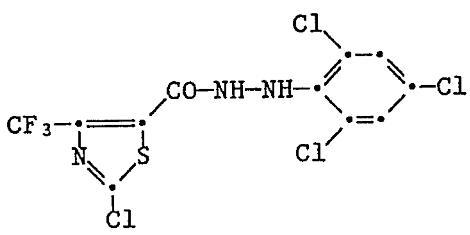

Eine Lösung von 9.3 g 2-Chlor-4-trifluormethyl-thiazol-5-carbonsäurechlorid (0.037 Mole) in 50 ml Acetonitril wird vorgelegt und dazu eine Lösung von 8.3 g 2,4,6-Trichlor-phenylhydrazin 95 % (0.037 Mole) und 3.8 g Triäthylamin (0.037 Mole) in 50 ml Acetonitril bei 0° dazugetropft. Die entstandene Suspension wird über Nacht bei Raumtemperatur weitergerührt, dann in 1 l Wasser gegeben. Es entstehen Kristalle, die abgenutscht und in Aethanol umkristallisiert werden. Schmelzpunkt 184 - 186°, Ausbeute 7.2 g (46 % d.Th.).

In analoger Weise zu diesem Beispiel werden die in der Tabelle 2 aufgeführten Thiazol-5-carbonsäure-hydrazide hergestellt.

Tabelle 2

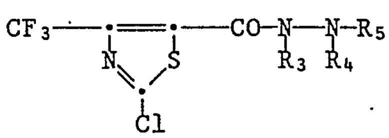

| Nr. | $R_3$ | $R_4$ | $R_5$ | phys. Daten |
|---|---|---|---|---|
| 2.001 | H | H | H | |
| 2.002 | H | $CH_3$ | $CH_3$ | |
| 2.003 | $CH_3$ | H | $CH_3$ | |
| 2.004 | H | H | Phenyl | Smp. 137-138° |
| 2.005 | H | H | CO-Phenyl | Smp. 192-194° |
| 2.006 | H | H | $SO_2$-Phenyl | |
| 2.007 | H | H | 2-Chlorphenyl | |
| 2.008 | H | H | 4-Chlorphenyl | |
| 2.009 | H | H | 2,4,6-Trichlorphenyl | Smp. 184-186° |

Formulierungsbeispiele für Wirkstoffe der Formel I oder Mischungen dieser Wirkstoffe mit Herbiziden

Beispiel 3: Spritzpulver

| | a) | b) | c) |
|---|---|---|---|
| Wirkstoff der Formel I oder Mischung mit Herbizid | 20 % | 60 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyäthylenglykoläther/7-8 Mol AeO) | - | 2 % | 2 % |
| Hochdisperse Kieselsäure | 5 % | 27 % | 27 % |
| Kaolin | 67 % | - | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

Beispiel 4: Emulsion-Konzentrat

| | a) | b) |
|---|---|---|
| Wirkstoff der Formel I oder Mischung mit Herbizid | 10 % | 1 % |
| Octylphenolpolyäthylenglykoläther (4-5 Mol AeO) | 3 % | 3 % |
| Ca-Dodecylbenzolsulfonat | 3 % | 3 % |
| Ricinusölpolyglykoläther (36 Mol AeO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

Beispiel 5: Stäubemittel

| | a) | b) |
|---|---|---|
| Wirkstoff der Formel I oder Mischung mit dem Herbizid der Formel I | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

Beispiel 6: Extruder Granulat

| | a) | b) |
|---|---|---|
| Wirkstoff der Formel I oder Mischung mit dem Herbizid der Formel V | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

Beispiel 7: Umhüllungs-Granulat

| | |
|---|---|
| Wirkstoff der Formel I oder Mischung mit dem Herbizid der Formel V | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

Beispiel 8: Suspensions-Konzentrat

| | a) | b) |
|---|---|---|
| Wirkstoff der Formel I oder Mischung mit dem Herbizid der Formel V | 40 % | 5 % |
| Aethylenglykol | 10 % | 10 % |
| Nonylphenolpolyäthylenglykoläther (15 MOl AeO) | 6 % | 1 % |
| Na-Ligninsulfonat | 10 % | 5 % |
| Carboxymethylcellulose | 1 % | 1 % |
| 37 %ige wässrige Formaldehyd-Lösung | 0,2 % | 0,2 % |
| Silikonöl in Form einer 75 %igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 9: Salzlösung

| | |
|---|---|
| Wirkstoff der Formel I oder Mischung mit dem Herbizid der Formel IV | 5 % |
| Isopropylamin | 1 % |
| Octylphenolpolyäthylenglykoläther (78 Mol AeO) | 3 % |
| Wasser | 91 %. |

Biologische Beispiele

Die Fähigkeit der Verbindungen der Formel I, Kulturpflanzen vor der phytotoxischen Wirkung starker Herbizide zu schützen, kann aus dem folgenden Beispiel ersehen werden. In der Versuchsbeschreibung werden die Verbindungen der Formel I als Safener oder Gegenmittel (Antidote) bezeichnet.

Beispiel 10:

Versuch mit Herbizid und Gegenmittel in Mais. Herbizid und Gegenmittel werden zusammen als Tankmischung im Vorauflaufverfahren appliziert.

Plastikcontainer (25 cm lang x 17 cm breit x 12 cm hoch) werden mit sandiger Lehmerde gefüllt und Maissamen der Sorte LG 5 eingesät. Nach dem Bedecken der Samen wird die als Safener zu prüfende Substanz zusammen mit dem Herbizid in verdünnter Lösung als Tankmischung auf die Bodenoberfläche gesprüht. 21 Tage nach der Applikation wird die Schutzwirkung des Safeners in Prozent ausgewertet. Als Referenz dienen dabei die mit dem Herbizid allein behandelten Pflanzen (keine Schutzwirkung) sowie die vollständig unbehandelte Kontrolle (100%ige Schutzwirkung).

Die geprüften Verbindungen verringerten die Schadwirkung des Herbizides auf ein Mass, das auf den Ernteertrag keinen Einfluss mehr hat.

Beispiel 11:

Versuch mit wassergesätem Reis Antidot und Herbizid werden als Tankmischung preemergent appliziert.

Reissamen werden während 44 Stunden bei 30 Grad Celsius in Wasser vorgequollen.

Dann werden Plastiktöpfe von 500 ml Inahlt bis zwei cm unter den Rand mit sandiger Lehmerde gefüllt. Das Herbizid N-Chloracetyl-N-(2-propoxyethyl) 2,6-diethylanilin wird in einer Konzentration von 250 g Wirkstoff pro Hektar zusammen mit der als Safener zu prüfenden Substanz in einer Konzentration von 1500 g Wirkstoff auf die Bodenoberfläche aufgesprüht.

Anschliessend werden die vorgequollenen Samen auf die sumpfige Bodenoberfläche gesät.

Die Erde wird in einem feuchten Zustand gehalten und der Wasserstand entsprechend dem Reiswachstum sukzessive erhöht. 18 Tage nach der Präparateapplikation wird die Schutzwirkung des Safeners in Prozent bonitiert. Als Basis dienen dabei die mit dem Herbizid allein behandelten Pflanzen sowie die vollständig unbehandelte Kontrolle. Die Schutzwirkung ist die Differenz des Schadens welch die mit dem Herbizid allein behandelten Pflanzen erleiden und dem den die mit Herbizid und Safener behandelten Pflanzen aufweisen. Die Resultate sind in der Tabelle 3 zusammengefasst:

Tabelle 3

| Antidot No. | Schutzwirkung % |
|---|---|
| - (Vergleich ohne Antidot) | 0 |
| 1.080 | 38 |
| 1.081 | 38 |
| 1.085 | 50 |
| 1.094 | 38 |

Beispiel 12:

Versuch mit Mais. Herbizid und Antidot werden als Tankmischung pre-emergent appliziert. Zur Ueberprüfung der Safeningwirkung werden Samen von Mais (Sorte LG-9) in Töpfen von 9 cm Diameter in Erde angezogen. Die Kultur der Pflanzen erfolgt im Gewächshaus unter angepassten Temperatur- und Lichtverhältnissen. Das Giessen resp. Düngen der Pflanzen erfolgt nach Bedarf.

Die Applikation der Safenersubstanz erfolgt als Tankmischung zusammen mit dem Herbizid mit einer Wasseraufwandmenge von 550 lt./ha im Vorauflauf (pre). Als Herbizid wurde N-(2-methoxy-1-methyläthyl)-N-chloracetyl-2-äthyl-6-methylanilin (1) in einer Aufwandmenge von 8 kg/ha verwendet. Das erfindungsgemässe Antidot wurde in einer Aufwandmenge von 1,5 kg/ha beigemischt. Zur Erfassung der Schutzwirkung wird nach 21 Tagen, die allgemeine Schädigung (Phytotox) an den Pflanzen bonitiert (= % Phyto; 0 % Phyto = kein Schaden, wie unbehandelte Kontrolle; 100 % Phyto = Totalschaden). Aus der Differenz der Schädigung der Herbizidbehandlung allein und der Kombination von Herbizid + Safener ergibt sich die in der Tabelle 4 aufgeführte Schutzwirkung in %.

Tabelle 4

| Antidot No. | Schutzwirkung % |
|---|---|
| 1.014 | 37,5 |
| 1.017 | 62,5 |
| 1.040 | 62,5 |
| 1.081 | 37,5 |
| 1.085 | 62,5 |
| 1.089 | 37,5 |
| 1.094 | 25 |

Beispiel 13:

Versuch mit Hirse und Mais. Herbizid und Antidot werden als Tankmischung pre-emergent appliziert.

Zur Ueberprüfung der Safeningwirkung werden Samen von Sorghum (Hirse der Sorte Funk G-623) und Mais (Sorte Blizzard) in Töpfen 11 cm Diameter in Erde angezogen. Die Kultur der Pflanzen erfolgt im Gewächshaus unter angepassten Temperatur- und Lichtverhältnissen. Das Giessen resp. Düngen der Pflanzen erfolgt nach Bedarf. Die Anzucht von Echinochloa (Unkraut) erfolgt nach dem gleichen Verfahren.

Die Applikation der Safenersubstanz erfolgt als Tankmischung zusammen mit dem Herbizid mit einer Wasseraufwandmenge von 550 lt./ha im Vorauflauf (pre). Als Herbizid dient N-(2-methoxy-1-methyläthyl)-N-chloracetyl-2-äthyl-6-methylanilin, Metolachlor (1) resp. N-(Isopropyl)-N-chloracetyl-3,3,5-trimethyl-cyclohex-1-enamid, Trimexachlor (2) in den angegebenen Aufwandmenge. Das erfindungsgemässe Antidot wird in derselben sowie 1/4 Aufwandmenge beigemischt.

Zur Erfassung der Schutzwirkung wird nach 21 Tagen, (Tabelle 5), die allgemeine Schädigung (Phytotox) and den Pflanzen bonitiert (0 % Phyto = kein Schaden, wie unbehandelte Kontrolle; 100 % Phyto = Totalschaden). Aus der Differenz der Phyto der Herbizidbehandlung allein und der Kombination von Herbizid + Safener ergibt sich die in der Tabelle 5 aufgeführte Schutzwirkung in %.

Tabelle 5

| Antidot No. | Aufwandmenge | Herbizid No. | Aufwandmenge | Pflanze | Schutzwirkung in % |
|---|---|---|---|---|---|
| 1.040 | 1000 g/ha | (1) | 1000 g/ha | Hirse | 35 |
| 1.040 | 1000 g/ha | (1) | 1000 g/ha | Echinochloa crus galli | 0 |
| 1.040 | 250 g/ha | (1) | 1000 g/ha | Hirse | 10 |
| 1.040 | 250 g/ha | (1) | 1000 g/ha | Echinochloa crus galli | 0 |
| 1.040 | 1000 g/ha | (2) | 1000 g/ha | Hirse | 40 |
| 1.040 | 1000 g/ha | (2) | 1000 g/ha | Echinochloa crus galli | 0 |
| 1.040 | 250 g/ha | (2) | 1000 g/ha | Hirse | 20 |
| 1.040 | 250 g/ha | (2) | 1000 g/ha | Echinochloa crus galli | 0 |
| 1.040 | 8000 g/ha | (1) | 8000 g/ha | Mais | 70 |
| 1.040 | 8000 g/ha | (1) | 8000 g/ha | Echinochloa crus galli | 0 |
| 1.040 | 2000 g/ha | (1) | 8000 g/ha | Mais | 60 |
| 1.040 | 2000 g/ha | (1) | 8000 g/ha | Echinochloa crus galli | 0 |
| 1.040 | 8000 g/ha | (2) | 8000 g/ha | Mais | 40 |
| 1.040 | 8000 g/ha | (2) | 8000 g/ha | Echinochloa crus galli | 0 |
| 1.040 | 2000 g/ha | (2) | 8000 g/ha | Mais | 35 |
| 1.040 | 2000 g/ha | (2) | 8000 g/ha | Echinochloa crus galli | 0 |

Beispiel 14:

Versuch in Hirse. Herbizid und Antidot werden pre-emergent als Tankmischung appliziert.

Zur Ueberprüfung der Safeningwirkung werden Samen von Sorghum (Sorte Funk G-623) in Töpfen von 9 cm Diameter in Erde angezogen. Die Kultur der Pflanzen erfolgt im Gewächshaus unter angepassten Temperatur- und Lichtverhältnissen. Das Giessen resp. Düngen der Pflanzen erfolgt nach Bedarf.

Die Applikation der Safenersubstanz erfolgt als Tankmischung zusammen mit dem Herbizid mit einer Wasseraufwandmenge von 550 lt./ha im Vorauflauf (pre). Als Herbizid wird N-(2-Methoxy-1-methyläthyl)-N-chloracetyl-2- äthyl-6-methylanilin (Metolachlor) in einer Aufwandmenge von 1,5 kg/ha. Das erfindungsgemässe Antidot wird ebenfalls in einer Aufwandmenge von 1,5 kg/ha dazugegeben.

Zur Erfassung der Schutzwirkung wird nach 21 Tagen, die allgemeine Schädigung (Phytotox) an den Pflanzen bonitiert (0 % Phyto = kein Schaden, wie unbehandelte Kontrolle; 100 % Phyto = Totalschaden). Aus der Differenz der Phyto der Herbizidbehandlung allein und der Kombination von Herbizid + Safener ergibt sich die in der Tabelle 6 aufgeführte Schutzwirkung in %.

Tabelle 6

| Antidot No. | Schutzwirkung % |
|---|---|
| 1.014 | 50 |
| 1.017 | 37,5 |
| 1.085 | 37,5 |

Beispiel 15:

Versuch mit gesätem Reis. Das Antidot wird durch Samenquellung auf die Samen appliziert, das Herbizid wird pre-emergent nach der Saat gespritzt.

Zur Ueberprüfung der Safeningwirkung werden Samen von Reis (Sorte S-201) in eine Wasserlösung mit Safenersubstanz während 48 Stunden vorgequollen (seed soaking). Die Inkubationszeit vor der Saat beträgt 24 Stunden und das Herbizid wird pre-emergent auf die Erde aufgesprüht.

Die Samen werden auf sumpfige Erde in Töpfe 9 x 9 cm gesät. Die Kultur der Pflanzen erfolgt im

Gewächshaus unter angepassten Temperatur- und Lichtverhältnissen. Das Giessen resp. Düngen der Pflanzen erfolgt nach Bedarf.

Die Applikation des Herbizids erfolgt mit einer Wasseraufwandmenge von 550 lt./ha im Vorauflauf (pre) gleich nach der Saat auf die vorgequollenen Samen. Als Herbizid wird N-(n-Propoxyäthyl)-N-chloracetyl-2,6-diäthylanilid, Pretilachlor (3) resp. N-(2-Methoxyäthylphenylsulfonyl)-N'-(4,6-dimethoxy-1,3,5-triazin-2-yl)-harnstoff (4) in der angebenen Aufwandmenge appliziert. Das erfindungsgemässe Antidot wird in einer wässrigen Lösung in der angegebenen Konzentration zur Samenquellung vorgegeben. Zur Erfassung der Schutzwirkung wird nach 26 Tagen, die allgemeine Schädigung (Phytotox) an den Pflanzen bonitiert (0 % Phyto = kein Schaden wie unbehandelte Kontrolle; 100 % Phyto = Totalschaden). Aus der Differenz der Phyto der Herbizidbehandlung allein und der Kombination von Herbizid + Safener ergibt sich die in der Tabelle 7 aufgeführte Schutzwirkung in %.

Tabelle 7

| Antidot No. | Aufwandmenge | Herbizid No. | Aufwandmenge | Schutzwirkung in % |
|---|---|---|---|---|
| 1.014 | 100 ppm | (3) | 250 g/ha | 37,5 |
| 1.017 | 100 ppm | (3) | 250 g/ha | 37,5 |
| 1.018 | 100 ppm | (3) | 250 g/ha | 62,5 |
| 1.040 | 100 ppm | (3) | 250 g/ha | 37,5 |
| 1.065 | 100 ppm | (3) | 250 g/ha | 62,5 |
| 1.040 | 150 ppm | (4) | 30 g/ha | 45 |
| 1.040 | 300 ppm | (4) | 30 g/ha | 55 |

Beispiel 16:

Versuch mit Reis. Antidot und Herbizid werden pre-emergent als Tankmischung appliziert.

Zur Ueberprüfung der Safeningwirkung werden Samen von Reis in Töpfen von 9 cm Diameter in Erde angezogen. Die Kultur der Pflanzen erfolgt im Gewächshaus unter angepassten Temperatur- und Lichtverhältnissen. Das Giessen resp. Düngen der Pflanzen erfolgt nach Bedarf.

Die Applikation der Safenersubstanz erfolgt als Tankmischung zusammen mit dem Herbizid mit einer Wasseraufwandmenge von 550 lt./ha im Vorauflauf (pre). Als Herbizid wird entweder N-(2-methoxy-1-methyläthyl)-N-chloracetyl-2-äthyl-6-methylanilid, Metolachlor (1), oder N-(2-Methoxyäthylphenylsulfonyl)-N'-(4,6-dimethoxy-1,3,5-triazin-2-yl)-harnstoff (4) in der gegebenen Aufwandmenge verwendet. Das erfindungsgemässe Antidot wird in einer Aufwandmenge von 1,5 kg/ha für Herbizid 1 resp. 30-60 g/ha für Herbizid 4 dazugemischt. Zur Erfassung der Schutzwirkung wird nach 21 Tagen, die allgemeine Schädigung (Phytotox) an den Pflanzen bonitiert (0 % Phyto = kein Schaden, wie unbehandelte Kontrolle; 100 % Phyto = Totalschaden). Aus der Differenz der Phyto der Herbizidbehandlung allein und der Kombination von Herbizid + Safener ergibt sich die in der Tabelle 8 aufgeführte Schutzwirkung in %.

Tabelle 8

| Antidot No. | Aufwandmenge | Herbizid No. | Aufwandmenge | Reis | Schutzwirkung in % |
|---|---|---|---|---|---|
| 1.017 | 1500 g/ha | (1) | 375 g/ha | Sorte Starbonet | 25 |
| 1.040 | 1500 g/ha | (1) | 375 g/ha | Sorte Starbonet | 12,5 |
| 1.040 | 60 g/ha | (4) | 30 g/ha | Sorte S-201 | 50 |
| 1.040 | 30 g/ha | (4) | 30 g/ha | Sorte S-201 | 35 |

Beispiel 17:

Versuch mit verschiedenen Reissorten und Unkräutern. Antidot und Herbizid werden als Tankmischung post-emergent appliziert.

Zur Ueberprüfung der Safeningwirkung werden Samen von 8 Reis-Sorten in Töpfen 11 cm Diameter in Erde angezogen. Die Kultur der Pflanzen erfolgt im Gewächshaus unter angepassten Temperatur- und Lichtverhältnissen. Das Giessen resp. Düngen der Pflanzen erfolgt nach Bedarf. Die Unkräuter Echinochloa, Rottboellia und Digitaria werden nach dem gleichen Verfahren angezogen.

Die Applikation der Safenersubstanz erfolgt als Tankmischung zusammen mit dem Herbizid mit einer Wasseraufwandmenge von 550 lt./ha im Nachauflauf (post). Als Herbizid werden N-(2-Methoxyäthylphenylsulfonyl)-N'-(4,6-dimethoxy-1,3,5-triazin-2-yl)-harnstoff (4) oder das 2R Enantiomere des Propinylesters der 2-[4-(5-Chlor-3-fluor-pyridin-2-yloxy)-phenoxy]-propionsäure (5) in der angegebenen Aufwandmenge auf die Pflänzchen gespritzt. Das Antidot No. 1.040 wird in der gegebenen Aufwandmenge dazugemischt. Zur Erfassung der Schutzwirkung wird 21 Tagen nach der Herbizidapplikation die allgemeine Schädigung

(Phytotox) an den Pflanzen bonitiert (0 % Phyto = kein Schaden, wie unbehandelte Kontrolle; 100 % Phyto = Totalschaden). Aus der Differenz der Phyto der Herbizidbehandlung allein und der Kombination von Herbizid + Safener ergibt sich die in der Tabelle 9 aufgeführte Schutzwirkung in %.

Tabelle 9

| Pflanze | Herbizid No. | Aufwandmenge | Antidot No. | Aufwandmenge | Schutzwirkung |
|---|---|---|---|---|---|
| Reis "S-201" | (4) | 30 g/ha | 1.040 | 60 g/ha | 55 % |
| Reis "S-201" | (4) | 30 g/ha | 1.040 | 30 g/ha | 60 % |
| Reis "New Bonet" | (5) | 60 g/ha | 1.040 | 120 g/ha | 30 % |
| Reis "New Bonet" | (5) | 60 g/ha | 1.040 | 30 g/ha | 15 % |
| Reis "Balilla" | (5) | 60 g/ha | 1.040 | 120 g/ha | 30 % |
| Reis "Balilla" | (5) | 60 g/ha | 1.040 | 30 g/ha | 30 % |
| Reis "Jung Weon" | (5) | 60 g/ha | 1.040 | 120 g/ha | 45 % |
| Reis "Jung Weon" | (5) | 60 g/ha | 1.040 | 30 g/ha | 35 % |
| Reis "PB-56" | (5) | 60 g/ha | 1.040 | 120 g/ha | 45 % |
| Reis "PB-56" | (5) | 60 g/ha | 1.040 | 30 g/ha | 45 % |
| Reis "IR-54" | (5) | 60 g/ha | 1.040 | 120 g/ha | 45 % |
| Reis "IR-54" | (5) | 60 g/ha | 1.040 | 30 g/ha | 35 % |
| Reis "Yamabiko" | (5) | 60 g/ha | 1.040 | 120 g/ha | 25 % |
| Reis "Yamabiko" | (5) | 60 g/ha | 1.040 | 30 g/ha | 25 % |
| Reis "Starbonet" | (5) | 60 g/ha | 1.040 | 120 g/ha | 40 % |
| Reis "Starbonet" | (5) | 60 g/ha | 1.040 | 60 g/ha | 45 % |
| Digitaria sanguinalis | (5) | 60 g/ha | 1.040 | 120 g/ha | 0 % |
| Digitaria sanguinalis | (5) | 60 g/ha | 1.040 | 30 g/ha | 0 % |
| Rottboellia exaltata | (5) | 60 g/ha | 1.040 | 120 g/ha | 0 % |
| Rottboellia exaltata | (5) | 60 g/ha | 1.040 | 30 g/ha | 0 % |
| Echinochloa crus galli | (5) | 60 g/ha | 1.040 | 120 g/ha | 0 % |
| Echinochloa crus galli | (5) | 60 g/ha | 1.040 | 30 g/ha | 0 % |

Beispiel 18:

Versuch mit Reis und Mais. Das Antidot wird durch Samenbeizung appliziert, das Herbizid pre-emergent auf die Saat gespritzt.

Zur Ueberprüfung der Safeningwirkung werden Reissamen der Sorte "starbonet" und Maissamen der Sorte "Blizzard" mit dem Antidot No. 1.040 in einem Glasbehälter durch Schütteln und Rotation gut zusammengemischt. Die Samen werden dann in Töpfen von 11 cm Durchmesser in Erde angezogen. Die Kultur der Pflanzen erfolgt im Gewächshaus unter angepassten Temperatur-und Lichtverhältnissen. Das Giessen resp. Düngen der Pflanzen erfolgt nach Bedarf.

Die Applikation des Herbizids erfolgt gleich nach der Saat mit einer Wasseraufwandmenge von 550 lt./ha im Vorauflauf (pre). Als Herbizid werden N-(n-Propoxyäthyl)-N-chloracetyl-2,6-diäthylanilin, Pretilachlor (3), N-(Isopropyl)-N-chloracetyl-3,3,5-trimethyl-cyclohex-1-enamid, Trimexachlor (2) und N-(2-Methoxyäthylphenylsulfonyl)-N′-(4,6-dimethoxy-1,3,5-triazin-2-yl)-har nstoff (4) und N-(Methoxymethyl-N-chloracetyl-2,6-diäthylanilin, Alachlor (6) in der gegebenen Aufwandmenge gespritzt. Zur Erfassung der Schutzwirkung wird nach 25 Tagen, die allgemeine Schädigung (Phytotox) an den Pflanzen bonitiert (0 % Phyto = kein Schaden, wie unbehandelte Kontrolle; 100 % Phyto = Totalschaden). Aus der Differenz der Phyto der Herbizidbehandlung allein und der Kombination von Herbizid + Safener ergibt sich die in der Tabelle 10 aufgeführte Schutzwirkung in %.

Tabelle 10

| Antidot No. | Aufwandmenge | | | Herbizid No. | Aufwandmenge | | Schutzwirkung in % |
|---|---|---|---|---|---|---|---|
| 1.040 | 0,5 | g/kg | Reis | (3) | 1 | kg/ha | 45 |
| 1.040 | 0,25 | g/kg | Reis | (3) | 1 | kg/ha | 40 |
| 1.040 | 0,5 | g/kg | Reis | (2) | 0,5 | kg/ha | 40 |
| 1.040 | 0,25 | g/kg | Reis | (2) | 0,5 | kg/ha | 35 |
| 1.040 | 0,5 | g/kg | Reis | (6) | 1 | kg/ha | 50 |
| 1.040 | 0,25 | g/kg | Reis | (6) | 1 | kg/ha | 45 |
| 1.040 | 1 | g/kg | Mais | (4) | 30 | g/ha | 45 |

Beispiel 19:

Versuch mit Mais. Das Antidot wird durch Samenbeizung appliziert, das Herbizid post-emergent auf die Pflanzen gespritzt.

Zur Ueberprüfung der Safeningwirkung werden Maissamen der Sorte "Blizzard" mit dem Antidot No. 1.040 in einem Glasbehälter durch Schütteln und Rotation gut zusammengemischt. Die Samen werden in Töpfen von 11 cm Durchmesser in Erde angezogen. Die Kultur der Pflanzen erfolgt im Gewächshaus unter angepassten Temperatur- und Lichtverhältnissen. Das Giessen resp. Düngen der Pflanzen erfolgt nach Bedarf.

Die Applikation des Herbizids erfolgt mit einer Wasseraufwandmenge von 550 lt./ha im Nachauflauf (post). Als Herbizid wird N-(2-Methoxyäthylphenylsulfonyl)-N′-(4,6-dimethoxy-1,3,5-triazin-2-yl)harnstoff (4) post-emergent auf die sich im 2-3-Blatt-Stadium befindlichen Maispflänzchen gespritzt. Zur Erfassung der Schutzwirkung wird nach 25 Tagen die allgemeine Schädigung (Phytotox) an den Pflanzen bonitiert (0 % Phyto = kein Schaden, wie unbehandelte Kontrolle; 100 % Phyto = Totalschaden). Aus der Differenz der Phyto der Herbizidbehandlung allein und der Kombination von Herbizid + Safener ergibt sich die in der Tabelle 11 aufgeführte Schutzwirkung in %.

Tabelle 11

| Antidot No. | Aufwandmenge | Herbizid No. | Aufwandmenge | Schutzwirkung in % |
|---|---|---|---|---|
| 1.040 | 1 g/kg Mais | (4) | 30 g/ha | 15 |

**Patentansprüche**

1. Mittel zum Schutz von Kulturpflanzen vor der phytotoxischen Wirkung von stark wirksamen Agrarchemikalien, dadurch gekennzeichnet, dass es als herbizid-antagonisierenden Wirkstoff ein Thiazol-5-carbonsäureamid der Formel I

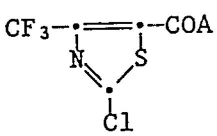

(I)

worin A einen Rest $-NR_1R_2$ oder $-NR_3-NR_4(CO)_mR_5$, $R_1$, $R_2$, $R_3$, $R_4$ und $R_5$ unabhängig voneinander je Wasserstoff, $C_1$-$C_8$Alkyl oder $C_3$-$C_8$Cycloalkyl, welches unsubstituiert oder durch $C_1$-$C_8$Alkoxy, $C_2$-$C_8$Alkoxyalkoxy, $C_1$-$C_8$Alkylthio, Cyano, einen Rest $-COOR_6$, $C_1$-$C_4$Alkylcarbamoyl, Di-$C_1$-$C_4$alkylcarbamoyl, $C_1$-$C_4$Alkylamino, Di-$C_1$-$C_4$Alkylamino, Piperidinocarbonyl, Pyrrolidinocarbonyl, Piperidino oder Pyrrolidino substituiert ist; ferner $C_3$-$C_8$Alkenyl oder $C_3$-$C_8$Cycloalkenyl, welches unsubstituiert oder substituiert ist durch Halogen, $C_1$-$C_8$ Alkoxy, $C_3$-$C_8$Cycloalkenyl, oder einen Rest Cyan, $-COOR_6$, $C_1$-$C_4$Alkylcarbamoyl, Di-$C_1$-$C_4$alkylcarbamoyl, U, Pyrrolidinocarbamoyl oder Piperidinocarbamoyl substituiert ist, ferner $C_3$-$C_8$Alkinyl, welches unsubstituiert oder durch U substituiert ist, ferner die Reste $-(E)_m$-U oder $-(E)_m$-Q; $R_1$ und $R_2$ resp. $R_4$ und $R_5$ zusammen mit dem Stickstoffatom, an das sie gebunden sind, bilden auch einen gesättigten oder ungesättigten 5 - 9 gliedrigen Heterocyclus, der ein- oder mehrfach durch Sauerstoff, Schwefel, Stickstoff, -NH-, $-NC_1$-$C_4$Alkyl, -CO- oder $-C(0R_7)0R_8$ und durch Halogen, Cyan, $C_1$-$C_8$Alkoxy, Amino, $C_1$-$C_4$Alkylamino, Di-$C_1$-$C_4$-Alkylamino, oder einen Rest $-COOR_6$ substituiert sein kann; $R_6$ ist Wasserstoff, $C_1$-$C_8$Alkyl, $C_3$-$C_8$Cycloalkyl, $C_3$-$C_8$Alkenyl, $C_3$-$C_8$Cycloalkenyl, $C_3$-$C_8$Alkinyl, $C_2$-$C_8$Alkoxyalkyl, $C_4$-$C_8$Alkoxyalkoxyalkyl, $C_1$-$C_4$Halogenalkyl, $-(C_1$-$C_3Alkyl)_mU$, $-(C_1$-$C_3Alkyl)_mQ$, $C_1$-$C_4$Halogenalkoxy, $C_1$-$C_4$Halogenalkoxy $C_1$-$C_4$alkyl;$R_7$ und $R_8$ sind

unabhängig voneinander je $C_1$-$C_4$Alkyl oder $R_7$ und $R_8$ bilden zusammen eine 2 - 4 gliedrige Alkylenkette, U ist ein Phenyl- oder Naphthylrest, der unsubstituiert oder ein- oder mehrfach durch Halogen, $C_1$-$C_4$Alkyl, -Y-$C_1$-$C_4$Alkyl, $C_1$-$C_4$Halogenalkyl, $C_1$-$C_4$Halogenalkoxy, Cyano, Nitro, -COOH, -$COOR_7$, -$CONH_2$, -$CONHR_7$, -$CON(R_7)_2$, $SO_2NHR_7$, $SO_2N(R_7)_2$, $SO_2NH_2$, Pyrrolidino, Piperidino, Pyrrolidinocarbonyl oder Piperidinocarbonyl substituiert ist.

E ist eine $C_1$-$C_8$Alkylen, $C_2$-$C_8$Alkenylenkette, die unsubstituiert oder durch Halogen, $C_1$-$C_4$Alkoxy, $C_1$-$C_4$Alkylthio, $C_1$-$C_4$ Halogenalkoxy oder einen Rest -$CO(O)_mR_6$, -$(CO)_mNH_2$, -$(CO)_mNHR_7$, -$(CO)_mNR_7R_8$, m ist Null oder 1, -$(CO)_mQ$ substituiert und/oder durch ein Glied -CO- oder -$C(OR_7)OR_8$- unterbrochen ist,

Q ist ein gesättigter oder ungesättigter 5 - 12 gliedriger Heterocyclus, der 1 - 4 Heteroatome oder eine Sulfinyl- oder Sulfonylgruppe auch in Kombination mit 1 - 2 Heteroatomen enthalten kann, der durch eine oder zwei Carbonylgruppen unterbrochen und benzanneliert sein kann, bedeuten, zusammen mit inerten Trägerstoffen und Zusätzen.

2. Mittel zur selektiven Bekämpfung von Unkräutern in Kulturen von Nutzpflanzen, welches ein Herbizid und einen herbizid-antagonistischen Wirkstoff enthält, dadurch gekennzeichnet, dass es als Herbizid einen Sulfonylharnstoffderivat, ein Halogenacetanilid oder ein Aryloxyphenoxypropionsäurederivat und als herbizid-antagonistischen Wirkstoff ein Thiazol-5-carbonsäureamid gemäss Anspruch 1 enthält, zusammen mit inerten Trägerstoffen und Zusätzen.

3. Mittel zur selektiven Bekämpfung von Unkräutern in Kulturen von Nutzpflanzen, welches ein Herbizid und einen herbizid-antagonistischen Wirkstoff enthält, dadurch gekennzeichnet, dass es als Herbizid ein Sulfonylharnstoffderivat der Formel IV enthält

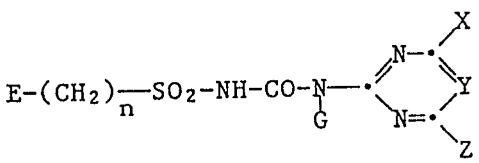

(IV)

worin

E eine Gruppe

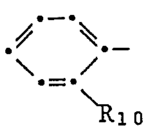 , 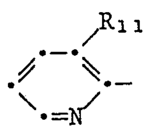 , 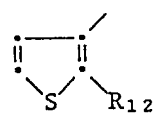 ,

oder 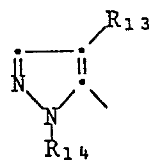 ,

n die Zahl null oder eins,

G Wasserstoff oder Methyl,

X Methoxy, Aethoxy, Difluormethoxy, Methyl oder Chlor

Y CH oder H,

Z Methoxy, Methyl, Difluormethoxy, Cyclopropyl oder Methylamino,

$R_{10}$ $C_2$-$C_5$-Alkoxyalkoxy, $C_1$-$C_4$-Halogenalkoxy, $C_1$-$C_4$-Halogenalkylthio, $C_2$-$C_4$-Halogenalkenyl, Chlor oder $C_1$-$C_4$-Alkoxycarbonyl,

$R_{11}$ Trifluormethyl oder Di($C_1$-$C_4$-alkyl)carbamoyl,

$R_{12}$ $C_1$-$C_4$-Alkoxycarbonyl,

$R_{13}$ $C_1$-$C_4$-Alkoxycarbonyl, und

$R_{14}$ $C_1$-$C_4$-Alkyl bedeuten, und als herbizid-antagonistischen Wirkstoff ein Thiazol-5-carbonsäureamid gemäss Anspruch 1 enthält, zusammen mit inerten Trägerstoffen und Zusätzen.

4. Mittel zur selektiven Bekämpfung von Unkräutern in Kulturen von Nutzpflanzen, welches ein Herbizid und einen Herbizid-antagonistischen Wirkstoff enthält, dadurch gekennzeichnet, dass es als Herbizid ein Halogenacetanilid der Formel V enthält

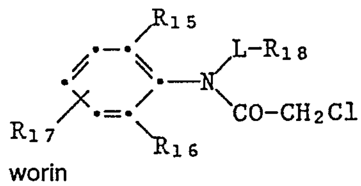

(V)

worin

L eine $C_1$-$C_4$-Alkylenbrücke,

$R_{15}$ $R_{16}$ und $R_{17}$ unabhängig voneinander Wasserstoff, Halogen, $C_1$-$C_4$-Alkyl, $C_1$-$C_4$-Alkoxy, $C_1$-$C_4$-Halogenalkyl, $C_2$-$C_5$-Alkoxyalkyl oder $C_2$-$C_5$-Alkylthioalkyl, und

$R_{18}$ $C_1$-$C_4$-Alkoxy, -COOH, $C_1$-$C_4$-Alkoxycarbonyl, $-CONH_2$, $C_1$-$C_4$-Alkylcarbamoyl, Di-$C_1$-$C_4$-alkylcarbamoyl, Cyan, $C_1$-$C_4$-Alkylcarbonyl,
gegebenenfalls substituiertes Benzoyl,
gegebenenfalls substituiertes Furyl,
gegebenenfalls substituiertes Thienyl,
gegebenenfalls substituiertes Pyrrolyl,
gegebenenfalls substituiertes Pyrazolyl,
gegebenenfalls substituiertes 1,3,4-Oxadiazol-2-yl,
gegebenenfalls substituiertes 1,3,4-Thiadiazol-2-yl,
gegebenenfalls substituiertes 1,2,4-Triazol-3-yl,
gegebenenfalls substituiertes Dioxolanyl,
gegebenenfalls substituiertes Dioxanyl,
gegebenenfalls substituiertes 1,3,4-Triazol-2-yl oder
gegebenenfalls substituiertes Tetrahydrofuryl, bedeutet, und als herbizid-antagonistischen Wirkstoff ein Thiazol-5-carbonsäureamid gemäss Anspruch 1 enthält, zusammen mit inerten Trägerstoffen und Zusätzen.

5. Mittel zur selektiven Bekämpfung von Unkräutern in Kulturen von Nutzpflanzen, welches ein Herbizid und einen Herbizid-antagonistischen Wirkstoff enthält, dadurch gekennzeichnet, dass es als Herbizid ein Aryloxyphenoxypropionsäurederivat der Formel IV enthält

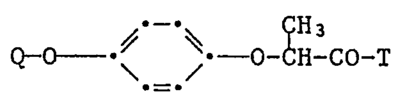

(VI)

worin

Q für den Rest

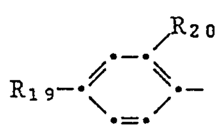

,

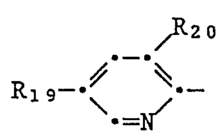

,

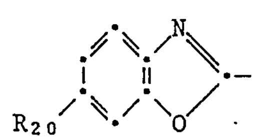

,

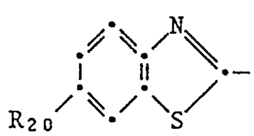

oder

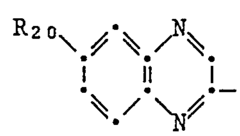

und T für

$-NR_{21}R_{22}$, $-N(CN)R_{21}$, $-OR_{22}$, $SR_{22}$ oder $-O-N=CR_{23}R_{22}$ stehen, wobei

$R_{19}$ Halogen oder Trifluormethyl,

$R_{20}$ Wasserstoff oder Halogen,

$R_{21}$ und $R_{22}$ unabhängig voneinander Wasserstoff, $C_1$-$C_8$-Alkoxy, $C_1$-$C_8$-Alkyl, Phenyl oder Benzyl, $R_{21}$ und $R_{22}$ zusammen mit dem sie tragenden Stickstoffatom einen 5- bis 6-gliedrigen gesättigten Stickstoffheterocyclus, der durch ein Sauerstoff- oder Schwefelatom unterbrochen sein kann,

$R_{23}$ $C_1$-$C_4$-Alkyl, $C_3$-$C_4$-Alkenyl, $C_3$-$C_4$-Alkinyl oder $C_2$-$C_4$-Alkoxyalkyl

$R_{24}$ Wasserstoff oder Aequivalent eines Alkalimetall-, Erdalkalimetall-, Kupfer- oder Eisen-Ions; einen quaternären $C_1$-$C_4$-Alkylammonium- oder $C_1$-$C_4$-Hydroxyalkylammonium-Rest; einen gegebenenfalls ein- oder mehrfach durch Amino, Halogen, Hydroxyl, Cyan, Nitro, Phenyl, $C_1$-$C_4$-Alkoxy, Polyäthoxy mit 2 bis 6 Aethylenoxideinheiten, $-COOR_{27}$, $-COSR_{27}$, $-CONH_2$, CON($C_1$-$C_4$-alkoxy)-$C_1$-$C_4$-alkyl, -CO-N-di-$C_1$-$C_4$-alkyl, CONH-$C_1$-$C_4$-alkyl, -N($C_1$-$C_4$-alkoxy)-$C_1$-$C_4$-alkyl oder Di-$C_1$-$C_4$-alkylamino substituierten $C_1$-$C_9$-Alkylrest; einen gegebenenfalls durch Halogen oder $C_1$-$C_4$-Alkoxy substituierten $C_3$-$C_9$-Alkenylrest; einen gegebenenfalls durch Halogen oder $C_1$-$C_4$-Alkoxy substituierten $C_3$-$C_9$-Alkinylrest;

$C_3$-$C_9$-Cycloalkyl; oder gegebenenfalls durch Cyan, $C_1$-$C_4$-Alkyl, $C_1$-$C_4$-Alkoxy, Acetyl, $-COOR_{27}$, $COSR_{27}$, $-CONH_2$, -CON($C_1$-$C_4$-alkoxy)-$C_1$-$C_4$-alkyl, -CO-N-di-$C_1$-$C_4$-alkyl oder -CONH-$C_1$-$C_4$-alkyl substituiertes Phenyl,
$R_{25}$ und $R_{26}$ unabhängig voneinander $C_1$-$C_4$-Alkyl oder zusammen eine 3- bis 6-gliedrige Alkylenkette und
$R_{27}$ Wasserstoff, $C_1$-$C_6$-Alkyl, $C_1$-$C_6$-Halogenalkyl, $C_2$-$C_6$-Alkoxyalkyl, $C_3$-$C_6$-Alkenyl, $C_3$-$C_6$-Halogenalkyl, $C_3$-$C_6$-Alkinyl oder $C_3$-$C_6$-Halogenalkinyl bedeuten, und als herbizid-antagonistischen Wirkstoff ein Thiazol-5-carbonsäureamid gemäss Anspruch 1 enthält, zusammen mit inerten Trägerstoffen und Zusätzen.

6. Mittel gemäss Ansprüchen 1 und 2, dadurch gekennzeichnet, dass es als herbizid-antagonistischen Wirkstoff ein Thiazol-5-carbonsäureamid, welches der Formel Ia entspricht, enthält

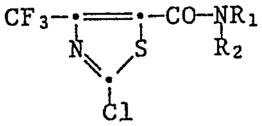
(Ia)

worin
$R_1$ und $R_2$ unabhängig voneinander je Wasserstoff, einen $C_1$-$C_8$Alkyl- oder $C_3$-$C_8$Cycloalkylrest, welcher unsubstituiert oder durch Halogen, Cyano, $C_1$-$C_4$Alkoxy oder $-COOR_6$ substituiert ist, einen $C_2$-$C_8$Alkenylrest, der unsubstituiert oder durch Halogen, Cyano, $C_1$-$C_4$Alkoxy oder $-COOR_6$ substituiert ist oder einen $C_3$-$C_8$Alkinylrest,
$R_2$ ferner einen Rest $-(E)_mU$ oder $-(E)_mQ$,
$R_1$ und $R_2$ zusammen eine 4 - 5 gliedrige Alkylenkette, die durch Sauerstoff, Schwefel, -NH- oder $-NR_7$ unterbrochen und/oder durch $C_1$-$C_4$Alkyl substituiert sein kann,
E $C_1$-$C_4$Alkylen, $C_3$-$C_4$-Cycloalkylen, $C_2$-$C_4$Alkenylen oder $C_2$-$C_4$Alkinylen, U Phenyl unsubstituiert oder durch Halogen, Cyano, $C_1$-$C_4$Alkoxy, $C_1$-$C_4$Haloalkyl, $C_1$-$C_4$Haloalkoxy, $COOR_6$, $-SO_2NH_2$, $-SO_2NHR_7$, $-CONH_2$, $-CONHR_7$, $-CON(R_7)_2$ substituiert,
Q einen 5 - 6 gliedrigen Heterocyclus der unsubstituiert oder durch Halogen, $C_1$-$C_4$Alkyl, $C_1$-$C_4$Alkoxy, $C_1$-$C_4$Haloalkyl oder $C_1$-$C_4$Haloalkoxy substituiert ist und $R_6$ und $R_7$ die unter der Formel I gegebene Bedeutung haben, bedeuten.

7. Mittel gemäss Ansprüchen 1 und 2, dadurch gekennzeichnet, dass es als herbizid-antagonisierenden Wirkstoff ein Thiazol-5-carbonsäureamid der Formel Ia enthält

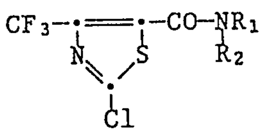
(Ia)

worin
$R_1$ Wasserstoff, $C_1$-$C_8$Alkyl oder $C_3$-$C_8$Alkinyl und $R_2$ $C_1$-$C_8$cyanoalkyl oder $C_3$-$C_8$cyanocycloalkyl, $C_3$-$C_8$Alkinyl, Phenyl, Halophenyl, $C_1$-$C_4$Alkylphenyl, $C_1$-$C_4$Alkylhalophenyl, Phenylamino, Halophenylamino oder Furyl bedeuten.

8. Mittel gemäss Ansprüchen 1 und 2, dadurch gekennzeichnet, dass es als herbizid-antagonisierenden Wirkstoff ein Thiazol-carbonsäure-hydrazid der Formel Ib enthält

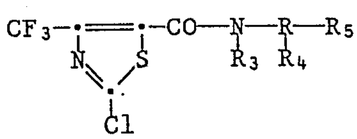
(Ib)

worin $R_3$, $R_4$ und $R_5$ unabhängig voneinander je Wasserstoff, $C_1$-$C_8$Alkyl, $C_3$-$C_8$Cycloalkyl, Phenyl oder Benzyl bedeuten, wobei der Phenylring unsubstituiert oder durch Halogen, $C_1$-$C_4$Alkyl, $C_1$-$C_4$ Haloalkyl, $C_1$-$C_4$Alkoxy, $C_1$-$C_4$Haloalkoxy, $-COOR_6$, $-CONH_2$, $-CONHR_7$, $-CON(R_7)_2$, $-SONH_2$, $-SONHR_7$, $-SO_2N(R_7)_2$, substituiert ist und
$R_4$ und $R_5$ bilden zusammen auch eine 4 - 5 gliedrige Alkylenkette, die durch Sauerstoff, Schwefel, -NH- oder $-NR_7$ unterbrochen und/oder durch $C_1$-$C_4$ Alkyl substituiert sein kann, bedeuten.

9. Mittel gemäss Ansprüchen 1 und 2, daduch gekennzeichnet, dass es als herbizid-antagonisierenden Wirkstoff ein Thiazol-5-carbonsäureamid enthält, ausgewählt aus
Thiazol-5-carbonsäure-N-methyl-N-benzylamid,
Thiazol-5-carbonsäure-benzylamid,
Thiazol-5-carbonsäure-N-methyl-N-(1-cyano-cyclopent-1-yl)-amid,
Thiazol-5-carbonsäure-N,N-di-propinylamid,
Thiazol-5-carbonsäure-N′-(2,4,6-trichlorphenyl)-hydrazid,
Thiazol-5-carbonsäure-furfurylamid,

Thiazol-5-carbonsäure-(4-amidosulfonyl)-anilid und
Thiazol-5-carbonsäure-N-methyl-N-(cyano-dimethylmethyl)-amid
Thiazol-5-carbonsäure-N-methyl-N-(2,6-dichlorbenzyl)-amid.

10. Verwendung eines Mittels gemäss Anspruch 1 zum Schützen von Kulturpflanzen vor der phytotoxischen Wirkung von Herbiziden.

11. Verfahren zur selektiven Bekämpfung von Unkräutern in Nutzpflanzenkulturen, dadurch gekennzeichnet, dass man die Kulturpflanzen oder deren Anbaufläche sowohl mit einer wirksamen Menge eines Herbizides als auch mit einer herbizid-antagonistisch wirksamen Menge eines Thiazol-5-carbonsäureamides gemäss Anspruch 1 als Gegenmittel behandelt.

12. Verfahren zum Schützen von Kulturpflanzen von Schäden, die bei der Applikation von Herbiziden auftreten, dadurch gekennzeichnet, dass man entweder die Anbaufläche für die Pflanze vor oder während der Applikation des Herbizides oder den Samen oder die Stecklinge an Pflanzen, oder die Pflanze selbst mit einer wirksamen Menge eines Thiazol-5-carbonsäureamides gemäss Anspruch 1 behandelt.

13. Saatgut von Nutzpflanzen, das mit einer herbizid-antagonistisch wirksamen Menge eines Thiazol-5-carbonsäureamides gemäss Anspruch 1 behandelt worden ist.

Europäisches Patentamt

# EUROPÄISCHER RECHERCHENBERICHT

Nummer der Anmeldung

EP 89 81 0171

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | GB-A-2 020 662 (MONSANTO CO)<br>* Ansprüche 1,12,13,40,43; Seiten 5,12, Tabelle I, Seite 17, Tabelle II, Seite 20, Zeilen 45,46, Seite 27, Tabelle VIII, jeweils Verbindung N,N-Diethyl-2-chlor-4-trifluormethyl-5-thiazolcarboxamid *; & US - A - 4199506 (Kat. D) | 1,2,4,6,10-13 | C 07 D 277/56<br>A 01 N 25/32 |
| D,A | US-A-4 251 261 (R.K. HOWE et al.)<br>* Anspruch 1 * | 1,2,4,10-13 | |
| D,A | EP-A-0 064 353 (MONSANTO CO)<br>* Ansprüche 1,2,8,14,18 * | 1,2,4,10-13 | |
| D,A | EP-A-0 044 201 (MONSANTO CO)<br>* Ansprüche 1,6,14,22,29,32 * | 1,2,4,10-13 | |
| D,A | EP-A-0 027 018 (MONSANTO CO)<br>* Zusammenfassung * | 1,6,10 | |
| P,D<br>A | EP-A-0 279 239 (CIBA-GEIGY AG)<br>* Ansprüche 1,15-18 * | 1,6-9 | **RECHERCHIERTE SACHGEBIETE (Int. Cl.4)** |
| A | EP-A-0 147 365 (CIBA-GEIGY AG)<br>* Ansprüche 1,5,15,20 * | 1-3,10-13 | A 01 N 25/00<br>A 01 N 47/00<br>C 07 D 277/00 |
| A | EP-A-0 127 469 (E.I. DU PONT DE NEMOURS & CO)<br>* Ansprüche 1,10,11,13,17 * | 1-3,10-13 | |

Der vorliegende Recherchenbericht wurde für alle Patentansprüche erstellt

| Recherchenort | Abschlußdatum der Recherche | Prüfer |
|---|---|---|
| BERLIN | 08-06-1989 | HASS C V F |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)